# EUROPEAN PATENT SPECIFICATION

(11) **EP 2 274 624 B1**
(45) Date of publication and mention of the grant of the patent: **09.11.2011**
(21) Application number: 09726947.6
(22) Date of filing: 31.03.2009
(51) Int. Cl.: G01N 33/74, G01N 33/543, G01N 33/58

(54) **PEGYLATED INSULIN-LIKE-GROWTH-FACTOR ASSAY**
ASSAYS MIT PEGYLIERTEN INSULINÄHNLICHEN WACHSTUMSFAKTOREN
TEST DE FACTEUR DE CROISSANCE ANALOGUE À L'INSULINE PÉGYLÉ

(30) Priority: 03.04.2008 EP 08006768
(43) Date of publication of application: 19.01.2011
(73) Proprietor: F. Hoffmann-La Roche AG, 4070 Basel (CH)
(72) Inventor: LANG, Kurt, 82377 Penzberg (DE); SCHAUBMAR, Andreas, 82377 Penzberg (DE); SCHLEYPEN, Julia, 81375 München (DE); SCHLOTHAUER, Tilman, 82377 Penzberg (DE)
(74) Representative: Burger, Alexander
(86) International application number: PCT/EP2009/002319
(87) International publication number: WO 2009/121551

(56) References cited:
- WO-A-99/46597
- WO-A-02/094853
- US-A1- 2002 192 718
- US-A1- 2004 014 156
- RAYMOND PHILIPPE ET AL: "Quantification of des-Arg-9-bradykinin using a chemiluminescence enzyme immunoassay: Application to its kinetic profile during plasma activation" JOURNAL OF IMMUNOLOGICAL METHODS, vol. 180, no. 2, 1995, pages 247-257, XP002494580 ISSN: 0022-1759

### SEQUENCE LISTING

<110> F. Hoffmann-La Roche AG
<120> PEGylated insulin-like-growth-factor assay
<130> 24884 WO
<150> EP 08006768.9
   <151> 2008-04-03
<160> 3
<170> PatentIn version 3.2
<210> 1
   <211> 70
   <212> PRT
   <213> Homo sapiens
<400> 1
   Gly Pro Glu Thr Leu Cys Gly Ala Glu Leu Val Asp Ala Leu Gln Phe
   1               5                   10                  15
   Val Cys Gly Asp Arg Gly Phe Tyr Phe Asn Lys Pro Thr Gly Tyr Gly
               20                  25                  30
   Ser Ser Ser Arg Arg Ala Pro Gln Thr Gly Ile Val Asp Glu Cys Cys
           35                  40                  45
   Phe Arg Ser Cys Asp Leu Arg Arg Leu Glu Met Tyr Cys Ala Pro Leu
       50                  55                  60
   Lys Pro Ala Lys Ser Ala
   65                  70
<210> 2
   <211> 67
   <212> PRT
   <213> Homo sapiens
<400> 2
   Ala Tyr Arg Pro Ser Glu Thr Leu Cys Gly Gly Glu Leu Val Asp Thr
   1               5                   10                  15
   Leu Gln Phe Val Cys Gly Asp Arg Gly Phe Tyr Phe Ser Arg Pro Ala
               20                  25                  30
   Ser Arg Val Ser Arg Arg Ser Arg Gly Ile Val Glu Glu Cys Cys Phe
           35                  40                  45
   Arg Ser Cys Asp Leu Ala Leu Leu Glu Thr Tyr Cys Ala Thr Pro Ala
       50                  55                  60
   Lys Ser Glu
   65
<210> 3
   <211> 237
   <212> PRT
   <213> Homo sapiens
<400> 3
   Asp Glu Ala Ile His Cys Pro Pro Cys Ser Glu Glu Lys Leu Ala Arg
   1               5                   10                  15
   Cys Arg Pro Pro Val Gly Cys Glu Glu Leu Val Arg Glu Pro Gly Cys
               20                  25                  30
   Gly Cys Cys Ala Thr Cys Ala Leu Gly Leu Gly Met Pro Cys Gly Val
           35                  40                  45
   Tyr Thr Pro Arg Cys Gly Ser Gly Leu Arg Cys Tyr Pro Pro Arg Gly
       50                  55                  60
   Val Glu Lys Pro Leu His Thr Leu Met His Gly Gln Gly Val Cys Met
   65                  70                  75                  80
   Glu Leu Ala Glu Ile Glu Ala Ile Gln Glu Ser Leu Gln Pro Ser Asp
                   85                  90                  95
   Lys Asp Glu Gly Asp His Pro Asn Asn Ser Phe Ser Pro Cys Ser Ala
               100                 105                 110
   His Asp Arg Arg Cys Leu Gln Lys His Phe Ala Lys Ile Arg Asp Arg
           115                 120                 125
   Ser Thr Ser Gly Gly Lys Met Lys Val Asn Gly Ala Pro Arg Glu Asp
       130                 135                 140
   Ala Arg Pro Val Pro Gln Gly Ser Cys Gln Ser Glu Leu His Arg Ala
   145                 150                 155                 160
   Leu Glu Arg Leu Ala Ala Ser Gln Ser Arg Thr His Glu Asp Leu Tyr
                   165                 170                 175
   Ile Ile Pro Ile Pro Asn Cys Asp Arg Asn Gly Asn Phe His Pro Lys
               180                 185                 190
   Gln Cys His Pro Ala Leu Asp Gly Gln Arg Gly Lys Cys Trp Cys Val
           195                 200                 205
   Asp Arg Lys Thr Gly Val Lys Leu Pro Gly Gly Leu Glu Pro Lys Gly
       210                 215                 220
   Glu Leu Asp Cys His Gln Leu Ala Asp Ser Phe Arg Glu
   225                 230                 235

## Description

The current invention is in the field of immunoassays, more precisely it is reported an immunoassay for the detection and quantification of PEGylated insulin-like-growth-factor by the formation and determination of a complex of PEGylated insulin-like-growth-factor and insulin-like-growth-factor-binding-protein.

### Background of the Invention

Insulin-like-growth-factors I and II (IGF I and IGF II) are members of the insulin superfamily of hormones, growth factors and neuropeptides whose biological actions are achieved through binding to cell surface receptors, e.g. the insulin-like-growth-factor I receptor or the insulin-like-growth-factor II receptor. The insulin-like-growth-factor and growth hormone (GH) axis plays a large part in regulating fetal and childhood somatic growth. Several decades of basic and clinical research have demonstrated that it also is critical in maintaining neoplastic growth (Khandwala, H.M., et al., Endocr. Rev. 21 (2000) 215-244). Insulin-like-growth-factor actions are regulated by insulin-like-growth-factor-binding-proteins (IGFBPs) that act as transporters of insulin-like-growth-factors, protect them from degradation, limit or inhibit their binding to receptors, and maintain a "reservoir" of biologically inactive insulin-like-growth-factor (Martin, J.L., and Baxter, R.C., IGF binding proteins as modulators of IGF actions, in Rosenfeld, R.G., and Roberts, C.T. (eds.), The IGF system, Molecular Biology, Physiology, and Clinical Applications (1999), Humana Press, Totowa, 227-255; Jones, J.L., and Clemmons, D.R., Endocr. Rev. 12 (1995) 10-21; Khandwala, H.M., et al., Endocr. Rev. 21 (2000) 215-244; Hwa, V., et al., The IGF binding protein superfamily, in Rosenfeld, R.G., and Roberts, C.T. (eds.), The IGF system, Molecular Biology, Physiology, and Clinical Applications (1999), Humana Press, Totowa, pp. 315-327). Virtually every level of the insulin-like-growth-factor system mediated response on the tumor tissues (IGFs, IGFBPs, IGF receptors) can be targeted for therapeutic approaches (Khandwala, H.M., et al., Endocr. Rev. 21 (2000) 215-244; Fanayan, S., et al., J. Biol. Chem. 275 (2000) 39146-39151; Imai, Y., et al., J. Biol. Chem. 275 (2000) 18188-18194). It should also be mentioned here that insulin-like-growth-factor-binding-protein-3 has insulin-like-growth-factor-independent anti-proliferative and proapoptotic effects (Wetterau, L.A., et al., Mol. Gen. Metab. 68 (1999) 161-181; Butt, A.J., et al., J. Biol. Chem. 275 (2000) 39174-39181). Human insulin-like-growth-factor I is a circulating hormone structurally related to insulin. Insulin-like-growth-factor I is traditionally considered the major mediator of the actions of growth hormone on peripheral tissues. Insulin-like-growth-factor I consists of 70 amino acids and is also named Somatomedin C and defined by SwissProt No. P01343. Use, activity and production are mentioned in, e.g., le Bouc, Y., et al., FEBS Lett. 196 (1986) 108-112; de Pagter-Holthuizen, P., et al., FEBS Lett. 195 (1986) 179-184; Sandberg Nordqvist, A.C., et al., Brain Res. Mol. Brain Res. 12 (1992) 275-277; Steenbergh, P.H., et al., Biochem. Biophys. Res. Commun. 175 (1991) 507-514; Tanner, J.M., et al., Acta Endocrinol. (Copenhagen) 84 (1977) 681-696; Uthne, K., et al., J. Clin. Endocrinol. Metab. 39 (1974) 548-554; EP 0 123 228; EP 0 128 733; US 5,861,373; US 5,714,460; EP 0 597 033; WO 02/32449; WO 93/02695.

The regulation of insulin-like-growth-factor I function is quite complex. In the circulation, only a marginal level of 0.2 % to 1.0 % of insulin-like-growth-factor I exist in the free form whereas the majority is bound to insulin-like-growth-factor-binding-proteins, which have very high affinities to insulin-like-growth-factors and modulate insulin-like-growth-factor I function. The factor can be locally liberated by mechanisms releasing insulin-like-growth-factor I such as proteolysis of insulin-like-growth-factor-binding-proteins by proteases.

Insulin-like-growth-factor I plays a paracrine role in the developing and mature brain (Werther, G.A., et al., Mol. Endocrinol. 4 (1990) 773-778). In vitro studies indicate that insulin-like-growth-factor I is a potent non-selective tropic agent for several types of neurons in the CNS (Knusel, B., et al., J. Neurosci. 10(1990) 558-570; Svrzic, D., and Schubert, D., Biochem. Biophys. Res. Commun. 172 (1990) 54-60), including dopaminergic neurons (Knusel, B., et al., J. Neurosci. 10 (1990) 558-570) and oligodendrocytes (McMorris, F.A., and Dubois-Dalcq, M., J. Neurosci. Res. 21 (1988) 199-209; McMorris, F.A., et al., Proc. Natl. Acad. Sci. USA 83 (1986) 822-826; Mozell, R.L., and McMorris, F.A., J. Neurosci. Res. 30 (1991) 382-390)). US 5,093,317 mentions that the survival of cholinergic neuronal cells is enhanced by administration of insulin-like-growth-factor II. It is further known that insulin-like-growth-factor I stimulates peripheral nerve regeneration (Kanje, M., et al., Brain Res. 486 (1989) 396-398) and enhance ornithine decarboxylase activity (US 5,093,317). US 5,861,373 and WO 93/02695 mention a method of treating injuries to or diseases of the central nervous system that predominantly affects glia and/or non-cholinergic neuronal cells by increasing the active concentration(s) of insulin-like-growth-factor I and/or analogues thereof in the central nervous system of the patient. WO 02/32449 is directed to methods for reducing or preventing ischemic damage in the central nervous system of a mammal by administering to the nasal cavity of the mammal a pharmaceutical composition comprising a therapeutically effective amount of insulin-like-growth-factor I or biologically active variants thereof. Insulin-like-growth-factor I is absorbed through the nasal cavity and transported into the central nervous system of the mammal in an amount effective to reduce or prevent ischemic damage associated with an ischemic event. In EP 0 874 641 the use of an insulin-like-growth-factor I or an insulin-like-growth-factor II for the manufacture of a medicament for treating or preventing neuronal damage in the central nervous system is reported.

Reduction of brain and serum levels of free insulin-like-growth-factor I have been related to the pathogenesis of sporadic and familial forms of Alzheimer's disease. Furthermore, insulin-like-growth-factor I protects neurons against Aβ-induced neurotoxicity (Niikura, T., et al., J. Neurosci. 21 (2001) 1902-1910; Dore, S., et al., Proc. Natl. Acad. Sci. USA 94 (1997) 4772-4777; Dore, S., et al., Ann. NY Acad. Sci. 890 (1999) 356-364). Recently, it was shown that peripherally administered insulin-like-growth-factor II is capable of reducing brain Aβ levels in rats and mice (Carro, E., et al., Nat. Med. 8 (2002) 1390-1397). Furthermore, the study demonstrated that in a transgenic AD mouse model prolonged insulin-like-growth-factor I treatment significantly reduced brain amyloid plaque load. These data strongly support the idea that insulin-like-growth-factor I is able to reduce brain Aβ levels and plaque-associated brain dementia by clearing Aβ from the brain.

Insulin-like-growth-factor I and insulin-like-growth-factor II are 67 % identical single chain polypeptides of 70 and 67 amino acids, respectively, sharing with insulin about 40 % sequence identity and presumed structural homology. The first 29 residues of insulin-like-growth-factors are homologous to the B-chain of insulin (B region, 1-29), followed by 12 residues that are analogous to the C-peptide of proinsulin (C region, 30-41), and a 21-residue region that is homologous to the A-chain of insulin (A region, 42-62). The carboxy-terminal octapeptide (D region, 63-70) has no counterpart in insulin and proinsulin (Murray-Rust, J., et al., BioEssays 14 (1992) 325-331; Baxter, R.C., et al., J. Biol. Chem. 267 (1992) 60-65). The insulin-like-growth-factors are the only members of the insulin superfamily in which the C region is not removed proteolytically after translation.

Insulin-like-growth-factor-binding-proteins (insulin-like-growth-factor-binding-proteins-1 to -6) are proteins of 216 to 289 residues, with e.g. mature insulin-like-growth-factor-binding-protein-5 consisting of 252 residues (Wetterau, L.A., et al., Mol. Gen. Metab. 68 (1999) 161-181; for review see e.g. Rajaram, S., et al., Endocr. Rev. 18 (1997) 801-831). All insulin-like-growth-factor-binding-proteins share a common domain organization. The highest conservation is found in the N-terminal (residues 1 to ca. 100) and C-terminal (from residue 170) cysteine rich domains. Twelve conserved cysteines are found in the N-tenninal domain and six in the C-terminal domain. The central, weakly conserved part (L-domain) contains most of the cleavage sites for specific proteases (Chernausek, S.D., et al., J. Biol. Chem. 270 (1995) 11377-11382). Several different fragments of insulin-like-growth-factor-binding-proteins have been described and biochemically characterized so far (Mazerbourg, S., et al., Endocrinology 140 (1999) 4175-4184). Mutagenesis studies suggest that the high affinity insulin-like-growth-factor binding site is located in the N-terminal domain (Wetterau, L.A., et al., Mol. Gen. Metab. 68 (1999) 161-181; Chemausek, S.D., et al., J. Biol. Chem. 270 (1995) 11377-11382) and that at least insulin-like-growth-factor-binding-protein-3 and insulin-like-growth-factor-binding-protein-2 contain two binding determinants, one in the N- and one at the C-terminal domains (Wetterau, L.A., et al., Mol. Gen. Metab. 68 (1999) 161-181). Recently, a group of insulin-like-growth-factor-binding-protein-related-proteins (IGFBP-rPs) which bind insulin-like-growth-factors with lower affinity than insulin-like-growth-factor-binding-proteins have been described (Hwa, V., et al., The IGF binding protein superfamily in Rosenfeld, R.G., and Roberts, C.T. (eds.), The IGF system, Molecular Biology, Physiology, and Clinical Applications (1999), Humana Press, Totowa, pp. 315-327). Insulin-like-growth-factor-binding-proteins and IGFBP-rPs share the highly conserved and cysteine-rich N-terminal domain which appears to be crucial for several biological actions, including their binding to insulin-like-growth-factors and high affinity binding to insulin (Hwa et al., 1999). N-terminal fragments of insulin-like-growth-factor-binding-protein-3, generated for example by plasma digestion, also bind insulin and physiologically are thus likely relevant for insulin action. Beyond the N-terminal domain, there is a lack of sequence similarity between the insulin-like-growth-factor-binding-proteins and IGFBP-rPs.

### Summary of the Invention

The first aspect of the current invention is an immunoassay for the detection of PEGylated insulin-like-growth-factor comprising a capture antibody and a tracer antibody, wherein said capture antibody is a monoclonal anti-(polyethylene glycol) antibody, said tracer antibody is a monoclonal anti-digoxygenin antibody, and said PEGylated insulin-like-growth-factor is detected as a complex with a digoxygenylated insulin-like-growth-factor-binding-protein, whereby the incubation step of said PEGylated insulin-like-growth-factor and said digoxygenylated insulin-like-growth-factor-binding-protein is for 12 to 24 hours at room temperature with a concentration of said digoxygenylated insulin-like-growth-factor-binding-protein of 5.0 µg/ml or less.

In one embodiment said anti-(polyethylene glycol) antibody is conjugated to a solid phase and said anti-digoxygenin antibody is conjugated to a detectable label. In another embodiment said conjugation is a chemical conjugation. In a further embodiment said detectable label is selected from enzymes, antigens, fluorescent groups, chemoluminescent groups and metal chelate complexes. In still a further embodiment said PEGylated insulin-like-growth-factor is an insulin-like-growth-factor I of SEQ ID NO: 1 or a PEGylated variant thereof. In a further embodiment said PEGylated insulin-like-growth-factor is mono-PEGylated. In still another embodiment said insulin-like-growth-factor-binding-protein is insulin-like-growth-factor-binding-protein-3, insulin-like-growth-factor-binding-protein-4, or insulin-like-growth-factor-binding-protein-5. In a further embodiment the immunoassay according to the invention is characterized in that the incubation step of said PEGylated insulin-like-growth-factor and said digoxygenylated insulin-like-growth-factor-binding-protein is of from 18 to 22 hours, preferably 20 hours. In still a further embodiment the immunoassay according to the invention is characterized in that the incubation step of said PEGylated insulin-like-growth-factor and said digoxygenylated insulin-like-growth-factor-binding-protein is with a concentration of said digoxygenylated insulin-like-growth-factor-binding-protein of from 0.1 to 5.0 µg/ml, or of from 0.1 µg/ml to 1.0 µg/ml.

The second aspect of the current invention is a method for the determination of PEGylated insulin-like-growth-factor in a sample comprising the following steps:
a) providing a sample to be analyzed,
b) incubating an anti-(polyethylene glycol) antibody conjugated to a solid phase with said sample to form an anti-(polyethylene glycol) antibody/PEGylated insulin-like-growth-factor-complex,
c) incubating said complex formed in b) with digoxygenylated insulin-like-growth-factor-binding-protein-4 to form a second complex comprising the complex formed in b) at room temperature for 12 to 24 hours with a concentration of said digoxygenylated insulin-like-growth-factor-binding-protein-4 of 5.0 µg/ml or less,
d) incubating said complex formed in c) with a horseradish peroxidase conjugated anti-digoxygenin antibody to form a third complex comprising the complex formed in c),
e) determining PEGylated insulin-like-growth-factor by incubating the complex formed in d) with ABTS and by detection of the formation of a colored product.

In one embodiment of said method a washing step is performed after steps b), and/or c), and/or d). In one embodiment said PEGylated insulin-like-growth-factor is PEGylated insulin-like-growth-factor-I or a PEGylated variant thereof. In another embodiment the incubation step of said PEGylated insulin-like-growth-factor and said digoxygenylated insulin-like-growth-factor-binding-protein-4 is for 18 to 22 hours. In a further embodiment the incubation step of said PEGylated insulin-like-growth-factor and said digoxygenylated insulin-like-growth-factor-binding-protein-4 is with a concentration of said digoxygenylated insulin-like-growth-factor-binding-protein-4 of from 0.1 µg/ml to 5.0 µg/ml.

A third aspect of the current invention is a method for the quantitative determination of the amount of PEGylated insulin-like-growth-factor I or a PEGylated variant thereof in a sample comprising the following steps:
a) providing a sample to be analyzed,
b) providing at least two reference samples each containing a defined but different amount of PEGylated insulin-like-growth-factor I,
c) incubating separately an anti-(polyethylene glycol) antibody conjugated to a solid phase with said sample and with said at least two reference samples containing different amounts of PEGylated insulin-like-growth-factor I to form an anti-(polyethylene glycol) antibody/PEGylated insulin-like-growth-factor-complex,
d) incubating separately said complex formed in c) in each of the sample and reference samples with digoxygenylated insulin-like-growth-factor-binding-protein-4 to form a second complex comprising the complex formed in c), whereby the incubating with digoxygenylated insulin-like-growth-factor-binding-protein-4 is for 12 to 24 hours at room temperature with a concentration of said digoxygenylated insulin-like-growth-factor-binding-protein-4 of 5.0 µg/ml or less,
e) incubating separately said complex formed in d) in each of the sample and reference samples with a horseradish peroxidase conjugated anti-digoxygenin antibody to form a third complex comprising the complex formed in d),
f) incubating separately the complex formed in e) in each of the sample and reference samples with ABTS for 5 to 15 minutes and determining the amount of the formed colored product,
g) quantitatively determining the amount of PEGylated insulin-like-growth-factor I or of a PEGylated variant thereof in said sample with a calibration curve calculated based on the amount of the formed colored product in the reference samples.

A fourth aspect of the current invention is the use of a method according to the invention for the follow-up of a patient to whom PEGylated insulin-like-growth-factor or a PEGylated variant thereof has been administered.

One embodiment of the aspects of the current specification is that said capture antibody is a mixture of said anti-(polyethylene glycol) antibody comprising at least two of said anti-(polyethylene glycol) antibodies that differ in the antibody site at which they are conjugated to the solid phase, and said tracer antibody is a mixture of said anti-digoxygenin antibody comprising at least two of said anti-digoxygenin antibodies that differ in the antibody site at which they are conjugated to the detectable label. In a further embodiment the conjugation of the antibody to its conjugation partner is performed by chemically binding via N-terminal and/or ε-amino groups (lysine), ε-amino groups of different lysines, carboxy-, sulfhydryl-, hydroxyl- and/or phenolic functional groups of the amino acid backbone of the antibody and/or sugar alcohol groups of the carbohydrate structure of the antibody. In one embodiment of the invention's aspects the capture antibody mixture or the tracer antibody mixture comprises the respective antibody conjugated via an amino group and via a carbohydrate structure to their conjugation partner. In a further embodiment the conjugation of the capture antibody to the solid phase is performed by passive adsorption, or via a specific binding pair. In one embodiment of the invention the specific binding pair (first component/second component) is selected from Streptavidin or Avidin/biotin, or antibody/antigen, or lectin/polysaccharide, or steroid/steroid binding protein, or hormone/hormone receptor, or enzyme/substrate, or IgG/Protein A and/or G. In another embodiment the capture antibody is conjugated to biotin and conjugation to the solid phase is performed via immobilized Avidin or Streptavidin. In another embodiment the capture antibody is an anti-(polyethylene glycol) antibody of the IgM class. In still another embodiment of the aspects of the invention is the tracer antibody conjugated to the detectable label via a specific binding pair. Another embodiment of the aspects of the current invention is that the ratio of capture antibody to tracer antibody is 1:10 to 50:1 (ratio means ratio of antibody molecules irrespective of the molecular weight of the conjugates which can be different).

### Detailed Description of the Invention

The current invention is directed to an immunoassay for the determination of PEGylated insulin-like-growth-factor or a PEGylated variant thereof by using a capture antibody and a tracer antibody, wherein said capture antibody is an anti-(polyethylene glycol) antibody and said tracer antibody is an anti-digoxygenin antibody, wherein said PEGylated insulin-like-growth-factor is determined as a complex formed between said PEGylated insulin-like-growth-factor and an insulin-like-growth-factor-binding-protein, whereby the incubation step of said PEGylated insulin-like-growth-factor and said digoxygenylated insulin-like-growth-factor-binding-protein is for 12 to 24 hours at room temperature with a concentration of said digoxygenylated insulin-like-growth-factor-binding-protein of 5.0 µg/ml or less.

Immunoassays are well known to the skilled artisan. Methods for carrying out such assays as well as practical applications and procedures are summarized in related textbooks. Examples of related textbooks are Tijssen, P., Preparation of enzyme-antibody or other enzyme-macromolecule conjugates (in: "Practice and theory of enzyme immunoassays" (1990), 221-278, Eds. R.H. Burdon and v. P.H. Knippenberg, Elsevier, Amsterdam) and various volumes of "Methods in Enzymology" (Eds. S.P. Colowick, N.O. Caplan, Academic Press), dealing with immunological detection methods, especially volumes 70, 73, 74, 84, 92 and 121.

Antibodies contain as proteins a number of reactive moieties, such as, for example, amino groups (lysines, alpha-amino groups), thiol groups (cystines, cysteine, and methionine), carboxylic acid groups (aspartic acid, glutamic acid) and sugar-alcoholic groups. These can be employed for coupling to a binding partner like a surface, a protein, a polymer (such as e.g. PEG, Cellulose or Polystyrol), an enzyme, or a member of a binding pair (see e.g. Aslam M., and Dent, A., Bioconjugation MacMillan Ref. Ltd. (1999) 50-100).

One of the most common reactive groups of proteins is the aliphatic ε-amine of the amino acid lysine. In general, nearly all antibodies contain abundant lysine. Lysine amines are reasonably good nucleophiles above pH 8.0 (pKa =9.18) and therefore react easily and cleanly with a variety of reagents to form stable bonds. Another common reactive group in antibodies is the thiol residue from the sulfur-containing amino acid cystine and its reduction product cysteine (or half cystine). Cysteine contains a free thiol group, which is more nucleophilic than amines and is generally the most reactive functional group in a protein. Thiols are generally reactive at neutral pH, and therefore can be coupled to other molecules selectively in the presence of amines. Since free sulfhydryl groups are relatively reactive, proteins with these groups often exist with them in their oxidized form as disulfide groups or disulfide bonds. In addition to cystine and cysteine, some proteins also have the amino acid methionine, which is containing sulfur in a thioether linkage. The literature reports the use of several thiolating crosslinking reagents such as Traut's reagent (2-iminothiolane), succinimidyl (acetylthio) acetate (SATA), or sulfosuccinimidyl 6-[3-(2-pyridyldithio) propionamido] hexanoate (Sulfo-LC-SPDP) to provide efficient ways of introducing multiple sulfhydryl groups via reactive amino groups. Reactive esters, particularly N-hydroxysuccinimide (NHS) esters, are among the most commonly employed reagents for modification of amine groups. The optimum pH for reaction in an aqueous environment is pH 8.0 to 9.0. Isothiocyanates are amine-modification reagents and form thiourea bonds with proteins. They react with protein amines in aqueous solution (optimally at pH 9.0 to 9.5). Aldehydes react under mild aqueous conditions with aliphatic and aromatic amines, hydrazines, and hydrazides to form an imine intermediate (Schiff's base). A Schiff's base can be selectively reduced with mild or strong reducing agents (such as sodium borohydride or sodium cyanoborohydride) to derive a stable alkyl amine bond. Other reagents that have been used to modify amines are acid anhydrides. For example, diethylenetriaminepentaacetic anhydride (DTPA) is a bifunctional chelating agent that contains two amine-reactive anhydride groups. It can react with N-terminal and ε-amine groups of proteins to form amide linkages. The anhydride ring opens to create multivalent, metal-chelating arms able to bind tightly to metals in a coordination complex.

Another common reactive group in antibodies are carboxylic acids (aspartic acid, glutamic acid). Proteins contain carboxylic acid groups at the C-terminal position and within the side chains of aspartic acid and glutamic acid. For conjugation is the carboxylic acid group usually converted to a reactive ester by the use of a water-soluble carbodiimide and reacted with a nucleophilic reagent such as an amine, hydrazide, or hydrazine. The amine-containing reagent should be weakly basic in order to react selectively with the activated carboxylic acid in the presence of other amines on the protein. Protein crosslinking can occur when the pH is raised above 8.0.

Sodium periodate can be used to oxidize the alcohol part of a sugar within a carbohydrate moiety to an aldehyde. Each aldehyde group can be reacted with an amine, hydrazide, or hydrazine as described for carboxylic acids. Since the carbohydrate moiety is predominantly found on the crystallizable fragment (Fc) region of an antibody, conjugation can be achieved through site-directed modification of the carbohydrate away from the antigen-binding site.

Thiol-reactive reagents are those that will couple to thiol groups on proteins, forming thioether-coupled products. These reagents react rapidly at slight acidic to neutral pH and therefore can be reacted selectively in the presence of amine groups. Haloacetyl derivatives, e.g. iodoacetamides, form thioether bonds and are reagents for thiol modification. In antibodies, the reaction takes place at cysteine groups that are either intrinsically present or that result from the reduction of cystine's disulfides at various positions of the antibody. Further useful reagents are maleimides. The reaction of maleimides with thiol-reactive reagents is essentially the same as with iodoacetamides. Maleimides react rapidly at slight acidic to neutral pH.

Amines, hydrazides, and hydrazines are aldehyde and carboxylic acid-reactive reagents (formation of amide, hydrazone, or alkyl amine bonds). Amines, hydrazides, and hydrazines can be coupled to carboxylic acids of proteins after the activation of the carboxyl group by a water-soluble carbodiimide. The amine-containing reagent must be weakly basic so that it reacts selectively with the carbodiimide-activated protein in the presence of the more highly basic ε-amines of lysine to form a stable amide bond. In the reaction with aldehyde groups, which can be generated on antibodies by periodate oxidation of the carbohydrate residues on the antibody, a Schiff's base intermediate is formed, which can be reduced to an alkyl amine through the reduction of the intermediate with sodium cyanoborohydride (mild and selective) or sodium borohydride (strong) water-soluble reducing agents.

The term "immunoassay" as used within the current invention denotes an immunological determination method, i.e. an in vitro method. With an immunoassay a direct determination of either the presence and/or the amount of PEGylated insulin-like-growth-factor or of a PEGylated variant thereof in a sample is possible (see e.g. The Immunoassay Handbook, edited by David Wild, M Stockton Press, 1994). In general comprise immunoassays one or more, in one embodiment two different, binding molecules specifically binding to the molecule to be analyzed in the sample. In one embodiment the immunoassay according to the invention comprises two different antibodies binding to different, non-overlapping epitopes on PEGylated insulin-like-growth-factor. In another embodiment the immunoassay according to the invention comprises one antibody specifically binding to PEGylated insulin-like-growth-factor or its PEGylated variant and one molecule binding to a non-overlapping epitope of the molecule to be detected. For detection purposes at least one of the binding molecules is labeled with a detectable label, e.g. including radioisotopes, enzymes, or dyes, which can be detected by radioactive disintegrations, enzymatically catalyzed color-production, fluorescence output or inhibition, or chemoluminescent output. Immunological determination methods include methods such as radioimmunoassay (RIA), enzyme-linked immunosorbent assay (ELISA), fluorescent immunoassay (FIA) and chemoluminescent assays (CLA). The immunoassay according to the current invention is in one embodiment a heterogeneous immunoassays. In such an assay it is possible to remove not bound molecules present in the sample to be analyzed from the complex comprising the capture antibody and the analyte, which is bound to a solid phase. The separation can be performed by centrifugation, filtration, magnetic separation or aspiration of the sample fluid from the solid phase, and is in one embodiment followed by repeated washing of the solid phase bound complex with a buffer. In one embodiment the immunoassay is a sandwich immunoassay (see e.g. Immunochemistry of Solid-Phase Immunoassay, John E. Butler, CRC Press, 1991). In this immunoassay the PEGylated insulin-like-growth-factor is in a first step bound to a solid phase immobilized antibody specifically binding to a first epitope of the PEGylated insulin-like-growth-factor. After the complex formation the sample is removed and the complex is repeatedly washed with a buffer. Afterwards a detection molecule binding to an epitope of the PEGylated insulin-like-growth-factor which is a non-overlapping epitope to the first epitope is added to the complex. Said detection molecule is generally conjugated to a detectable label, either directly (to e.g. a fluorescent group, a radiolabel, or a metal-chelate) or indirectly (to e.g. a first partner of a binding pair). The PEGylated insulin-like-growth-factor is "sandwiched" between the antibody and the detection molecule. A second wash step may be performed to remove unbound detection molecule. Finally the detectable label is detected with a suitable detection agent. In one embodiment of the immunoassay and method according to the invention the antibody binds to the (polyethylene glycol)-part and the detection molecule binds to the insulin-like-growth-factor-part of the PEGylated insulin-like-growth-factor or its PEGylated variant.

The term "sample" as used within this application denotes, but is not limited to, any quantity of a substance from a living thing or formerly living thing. Such living things include, but are not limited to, humans, mice, monkeys, rats, rabbits, and other animals. In one embodiment the sample in the immunoassay or method according to the invention is obtained from mouse, rat, dog, cynomolgus, or human. In another embodiment the sample in the immunoassay or method according to the invention is from cynomolgus or human. Such samples include, but are not limited to, whole blood, serum or plasma from an individual, which are the most widely used sources of sample in clinical routine.

The term "solid phase" as used within this application denotes a non-fluid substance, and includes particles (including microparticles and beads) made from materials such as polymer, metal (paramagnetic, ferromagnetic particles), glass, and ceramic; gel substances such as silica, alumina, and polymer gels; capillaries, which may be made of polymer, metal, glass, and/or ceramic; zeolites and other porous substances; electrodes; microtiter plates; solid strips; and cuvettes, tubes or other spectrometer sample containers. A solid phase component of an assay is distinguished from inert solid surfaces with which the assay may be in contact in that a "solid phase" contains at least one moiety on its surface, which is intended to interact with the capture molecule used in the assay. A solid phase may be a stationary component, such as a tube, strip, cuvette or microtiter plate, or may be non-stationary components, such as beads and microparticles. Microparticles can also be used as a solid phase for homogeneous assay formats. A variety of microparticles that allow both non-covalent or covalent attachment of proteins and other substances may be used. Such particles include polymer particles such as polystyrene and poly (methylmethacrylate); gold particles such as gold nanoparticles and gold colloids; and ceramic particles such as silica, glass, and metal oxide particles. See for example Martin, C.R., et al., Analytical Chemistry-News & Features (1998) 322A-327A. Solid supports for the immunoassays according to the invention are widely described in the state of the art (see, e.g., Butler, J.E., Methods 22 (2000) 4-23).

Chromogens (fluorescent or luminescent groups and dyes), enzymes, NMR-active groups or metal particles, haptens, e.g. digoxigenin, are examples of "detectable labels". The detectable label can also be a photoactivatable crosslinking group, e.g. an azido or an azirine group. A metal chelate which can be detected by electrochemoluminescence is in one embodiment the detectable label, with particular preference being given to ruthenium chelates, e.g. a ruthenium (bispyridyl)₃²⁺ chelate. Suitable ruthenium labeling groups are described, for example, in EP 0 580 979, WO 90/005301, WO 90/11511 and WO 92/14138.

For direct detection the labeling group can be selected from any known detectable group, such as dyes, luminescent labeling groups (such as chemoluminescent groups, e.g. acridinium esters or dioxetanes), or fluorescent dyes (e.g. fluorescein, coumarin, rhodamine, oxazine, resorufin, cyanine and derivatives thereof). Other examples of labeling groups are luminescent metal complexes (such as ruthenium or europium complexes), enzymes (e.g. as used for ELISA or for CEDIA (Cloned Enzyme Donor Immunoassay, e.g. EP-A-0 061 888)), and radioisotopes.

Indirect detection systems comprise, for example, that the detection reagent is labeled with a first partner of a bioaffine binding pair. Examples of suitable binding pairs are hapten or antigen/antibody, biotin or biotin analogues such as aminobiotin, iminobiotin or desthiobiotin/avidin or Streptavidin, sugar/lectin, nucleic acid or nucleic acid analogue/complementary nucleic acid, and receptor/ligand, e.g., steroid hormone receptor/steroid hormone. Preferred first binding pair members comprise hapten, antigen and hormone. Especially preferred are haptens like digoxin and biotin and analogues thereof. The second partner of such binding pair, e.g. an antibody, Streptavidin, etc., usually is labeled to allow for direct detection, e.g. by the labels as mentioned above.

In the immunological detection methods according to the current invention reagent conditions are chosen which allow for binding of the reagents employed, e.g. for binding of an antibody to PEGylated insulin-like-growth-factor. The skilled artisan refers to the result of such binding event by using the term "complex". The complex formed in an assay method according to the present invention either can be used to determine the presence or it can be used to determine the concentration, i.e. to quantify the amount.

The term "insulin-like-growth-factor" as used within this application denotes a protein of SEQ ID NO: 1 (insulin-like-growth-factor I) or SEQ ID NO: 2 (insulin-like-growth-factor II) or a variant thereof. A variant of an insulin-like-growth-factor is in one embodiment an insulin-like-growth-factor of SEQ ID NO: 1 with the lysine at position 27 substituted with a polar amino acid, and either the lysine at position 65 or the lysine at position 68 substituted with a polar amino acid. The term "polar amino acid" denotes arginine, glutamine, and asparagine, i.e. the lysine is substituted with arginine, glutamine, or asparagine. In one embodiment said polar amino acid is arginine. In another embodiment said PEGylated insulin-like-growth-factor is a mono-PEGylated insulin-like-growth-factor with the amino acid sequence of SEQ ID NO: 1 with the lysine at position 27 and 65 substituted with a polar amino acid and a PEG residue covalently bound to amino acid position 68. In a further embodiment said PEGylated insulin-like-growth-factor is a mono-PEGylated insulin-like-growth-factor with the amino acid sequence of SEQ ID NO: 1 with the lysine at position 27 and 68 substituted with a polar amino acid and a PEG residue covalently bound to amino acid position 65. In still a further embodiment said PEGylated insulin-like-growth-factor is a mono-PEGylated insulin-like-growth-factor with the amino acid sequence of SEQ ID NO: I with the lysine at position 27, or with the lysine at position 27 and 65 and/or 68 substituted with a polar amino acid and a PEG residue covalently bound to amino terminus of said factor.

The first aspect of the current invention is an immunoassay for the detection of a PEGylated insulin-like-growth-factor comprising a capture antibody, an insulin-like-growth-factor/insulin-like-growth-factor-binding-protein-complex, and a tracer antibody, wherein
a) said capture antibody is a monoclonal anti-(polyethylene glycol) antibody,
b) said PEGylated insulin-like-growth-factor is detected as a complex with a digoxygenylated insulin-like-growth-factor-binding-protein,
c) said tracer antibody is a monoclonal anti-digoxygenin antibody.

The antibody against polyethylene glycol (PEG) is biotinylated and in one embodiment bound to a solid phase, e.g. a Streptavidin coated microtiter plate. The PEGylated insulin-like-growth-factor or its PEGylated variant either as reference standard or from the test samples binds to the solid-phase conjugated anti-(polyethylene glycol) antibody in a first incubation step. The term "PEGylated insulin-like-growth-factor" as used within this application denotes an "insulin-like-growth-factor" to which a (polyethylene glycol) residue is covalently attached. Thereafter, the digoxygenylated detection reagent, in one embodiment a digoxygenylated insulin-like-growth-factor-binding-protein, which is present in excess, is binding in a second incubation step to the prior formed complex. In a mammal derived sample insulin-like-growth-factor will generally be complexed with endogenous insulin-like-growth-factor-binding-protein. In order to determine PEGylated insulin-like-growth-factor the endogenous insulin-like-growth-factor-binding-protein has to be replaced by the digoxygenylated insulin-like-growth-factor-binding-protein of the assay, which is therefore added in excess. Anti-digoxygenin antibody conjugated to horseradish peroxidase and ABTS-solution are used as detection system.

In one embodiment the capture antibody is a complete antibody, i.e. it comprises a light and a heavy chain whereby the light chain comprises a variable domain and a constant domain, and whereby the heavy chain comprises a variable domain, a C_{H}1, a C_{H}2, a C_{H}3, and optional a C_{H}4 domain as well as a hinge region. The capture antibody may in a different embodiment be selected from the light chain, the variable region of the heavy chain, a Fab, Fab', F(ab)₂, or F(ab')₂ fragment of said anti-(polyethylene glycol) antibody, i.e. it is either the light chain, the variable region of the heavy chain, the Fab, or Fab', or F(ab)₂, or F(ab')₂ fragment of said anti-(polyethylene glycol) antibody.

Depending on the amino acid sequence of the constant region of the heavy chain immunoglobulins are assigned to different classes: IgA, IgD, IgE, IgG, and IgM. Some of these classes are further divided into subclasses (isotypes), i.e. IgG in IgG1, IgG2, IgG3, and IgG4, or IgA in IgA1 and IgA2. In one embodiment the capture antibody is a multimeric antibody, e.g. an IgM.

The conjugation of a tracer and/or capture antibody to its conjugation partner can be performed by different methods, such as passive adsorption, chemical binding, or binding via a specific binding pair. The term "conjugation partner" as used herein denotes e.g. a solid phase, a polypeptide, a detectable label, or a member of a specific binding pair. In one embodiment the conjugation of the capture and/or tracer antibody to its conjugation partner is independently of each other performed by chemically binding via N-terminal and/or ε-amino groups (lysine), ε-amino groups of different lysines, carboxy-, sulfhydryl-, hydroxyl-, and/or phenolic functional groups of the amino acid backbone of the antibody, and/or sugar alcohol groups of the carbohydrate structure of the antibody. In one embodiment the capture and/or tracer antibody are/is conjugated to its conjugation partner via a specific binding pair. In one embodiment the capture antibody is conjugated to biotin and immobilization to a solid support is performed via solid phase immobilized Avidin or Streptavidin. In one embodiment the tracer antibody is conjugated to horseradish peroxidase and is an antibody against digoxygenin. The capture antibody is in another embodiment conjugated to the solid phase by passive adsorption. An antibody conjugated to the solid phase by passive adsorption comprises a mixture of antibodies conjugated to the solid phase via different antibody sites. Thus, the capture antibody conjugated to the solid phase by passive adsorption is a mixture of two or more different conjugates wherein the conjugates differ in the antibody site, i.e. the antibody amino acid residue, with which the conjugation to the solid phase is effected. Passive adsorption is, e. g., described by Butler, J.E., "Solid Phases in Immunoassay", page 205-225 in Diamandis, E.P. and Christopoulos, T.K. (Editors): Immunoassay (1996), Academic Press, San Diego. In one embodiment of the invention, the capture antibody is immobilized via a specific binding pair. Such a binding pair (first component/second component) is, for example, Streptavidin or Avidin/biotin, antibody/antigen (see, for example, Hermanson, G.T., et al., Bioconjugate Techniques, Academic Press, 1996), lectin/polysaccharide, steroid/steroid binding protein, hormone/hormone receptor, enzyme/substrate, IgG/Protein A and/or G and/or L, etc. In one embodiment the capture antibody is conjugated to biotin and immobilization is performed via immobilized Avidin or Streptavidin. In another embodiment the tracer antibody is conjugated to an electrochemiluminescent label, like a ruthenium bispyridyl complex.

The immunoassay according to the invention employs the specific interaction of insulin-like-growth-factor with insulin-like-growth-factor-binding-protein. Insulin-like-growth-factor-binding-protein specifically binds to insulin-like-growth-factor and the formed insulin-like-growth-factor/insulin-like-growth-factor-binding-protein-complex is detected. This complex cannot be detected directly and, thus, further binding partners are required. Therefore, the immunoassay according to the invention comprises as core elements:
a) a capture antibody specifically binding to insulin-like-growth-factor,
b) a tracer antibody specifically binding to insulin-like-growth-factor-binding-protein.

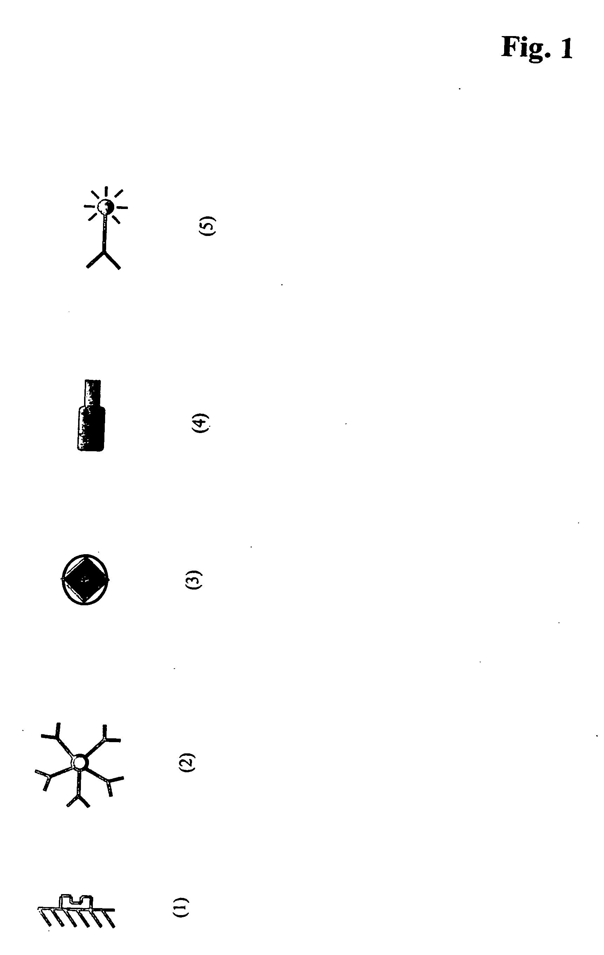

Thus, the immunoassay according to the invention for the detection of a PEGylated insulin-like-growth-factor comprises the following compounds (see also Figure 1):
- a solid phase,
- a capture antibody which is specifically binding to polyethylene glycol and which is conjugated to the solid phase,
- an insulin-like-growth-factor-binding-protein, which is conjugated either directly to a detectable label, or is conjugated to a first partner of a binding pair,
- optionally, if the insulin-like-growth-factor-binding-protein is conjugated to a first partner of a binding pair, a tracer molecule comprising the second partner of said binding pair.

The anti-(polyethylene glycol) antibody conjugated to the solid phase specifically binds to the polyethylene glycol residue of PEGylated compounds. If a sample containing PEGylated compounds is brought in contact with the anti-(polyethylene glycol) antibody conjugated to a solid phase, the PEGylated compounds will be bound by the anti-(polyethylene glycol) antibody and, thus, will be conjugated to the solid phase via the anti-(polyethylene glycol) antibody. The anti-(polyethylene glycol) antibody is in one embodiment a monoclonal antibody, and can be of any immunoglobulin class. In another embodiment said anti-(polyethylene glycol) antibody is a monoclonal anti-(polyethylene glycol) antibody of the IgM class. Exemplary anti-(polyethylene glycol) antibodies are reported in US 7,320,791 or WO 2002/094853. The conjugation of the anti-(polyethylene glycol) antibody to the solid phase can either be covalently or via a specific binding pair or via physical interactions.

The solid phase is in one embodiment a well of a micro titer plate. The conjugation of said capture anti-(polyethylene glycol) antibody to the solid phase is in one embodiment via a specific binding pair, e.g. via the specific binding pair Streptavidin/biotin, whereby the anti-(polyethylene glycol) antibody is linked to biotin via a covalent bond and the solid phase is linked to Streptavidin via a covalent bond.

The term "insulin-like-growth-factor-binding-protein" encompasses in the current invention the insulin-like-growth-factor-binding-proteins insulin-like-growth-factor-binding-protein-1, insulin-like-growth-factor-binding-protein-2, insulin-like-growth-factor-binding-protein-3, insulin-like-growth-factor-binding-protein-4, insulin-like-growth-factor-binding-protein-5, and insulin-like-growth-factor-binding-protein-6. The sequences of human insulin-like-growth-factor-binding-protein-1 to -6 are described in detail in the SwissProt Database (http://www.expasy.ch) and identified by the following Accession Nos.:

| **Name** | **Accession No.** |
|---|---|
| insulin-like-growth-factor-binding-protein-1 | P 08833 |
| insulin-like-growth-factor-binding-protein-2 | P 18065 |
| insulin-like-growth-factor-binding-protein-3 | P 17936 |
| insulin-like-growth-factor-binding-protein-4 | P 22692 |
| insulin-like-growth-factor-binding-protein-5 | P 24593 |
| insulin-like-growth-factor-binding-protein-6 | P 24592 |

The insulin-like-growth-factor-binding-protein in the immunoassay according to the invention is in one embodiment insulin-like-growth-factor-binding-protein-3, or insulin-like-growth-factor-binding-protein-4, or insulin-like-growth-factor-binding-protein-5.

In mammal derived samples, e.g. human samples, the insulin-like-growth-factor will be complexed with one of the endogenous insulin-like-growth-factor-binding-proteins-1 to -6, whereas insulin-like-growth-factor-protein-3 is the most abundant (Rajaram, S., et al., Endocr. Rev. 18 (1997) 801-831). In one embodiment the insulin-like-growth-factor-binding-protein in the immunoassay or method according to the invention is insulin-like-growth-factor-binding-protein-4 of SEQ ID NO: 3. The detectable label which is conjugated to the insulin-like-growth-factor-binding-protein is conjugated via a covalent bond. In one embodiment the detectable label is selected from enzymes, antigens, fluorescent groups, chemoluminescent groups, metal-chelate complexes, and electrochemiluminescent groups. In another embodiment the detectable label is selected from digoxygenin, and ruthenium bispyridyl complexes.

The next aspect of the current invention is a method for the determination of PEGylated insulin-like-growth-factor in a sample comprising the following steps:
a) providing a sample to be analyzed,
b) incubating an anti-(polyethylene glycol) antibody conjugated to a solid phase with said sample to form an anti-(polyethylene glycol) antibody/PEGylated insulin-like-growth-factor-complex,
c) incubating said complex formed in b) with digoxygenylated insulin-like-growth-factor-binding-protein-4 to form a complex comprising the complex formed in b),
d) incubating said complex formed in c) with a horseradish peroxidase conjugated anti-digoxygenin antibody to form a complex comprising the complex formed in c),
e) determining PEGylated insulin-like-growth-factor by incubating the complex formed in d) with ABTS and by the formation of a colored product.

In the determination of PEGylated insulin-like-growth-factor with the method according to the invention four steps are carried out. In the first step an anti-(polyethylene glycol) antibody, which is conjugated to a solid phase, e.g. via the specific binding pair Streptavidin/biotin, is incubated with a sample in question suspected to contain PEGylated polypeptides, especially to contain PEGylated insulin-like-growth-factor. In one embodiment the sample is blood serum from mouse, rat, dog, cynomolgus, or human. The first incubation step in one embodiment is of from 0.5 hours to 5 hours, e.g. about one hour. The anti-(polyethylene glycol) antibody specifically binds to PEGylated polypeptides contained in the sample and is thereby conjugating the PEGylated polypeptide also to the solid phase via the anti-(polyethylene glycol) antibody. After the first incubation step the solid phase is optionally washed with a buffered solution.

In the second incubation step the complex consisting of the solid phase conjugated anti-(polyethylene glycol) antibody and the PEGylated polypeptide formed in the first incubation step is incubated with digoxygenylated insulin-like-growth-factor-binding-protein-4. A further second complex is formed only if the PEGylated polypeptide contained in the complex obtained in the first incubation step is a PEGylated insulin-like-growth-factor. For the determination of PEGylated insulin-like-growth-factor an excess of insulin-like-growth-factor-binding-protein-4 is added in order to replace endogenous insulin-like-growth-factor-binding-protein complexed with the PEGylated insulin-like-growth-factor of the sample. The second complex consists of the solid-phase conjugated anti-(polyethylene glycol) antibody, the thereto bound PEGylated insulin-like-growth-factor and the thereto bound digoxygenylated insulin-like-growth-factor-binding-protein-4. The second incubation step in one embodiment is of from 12 to 24 hours, in another embodiment of from 18 to 22 hours. After the second incubation step the solid phase is optionally washed with a buffered solution.

In the third incubation step the complex consisting of the solid phase conjugated anti-(polyethylene glycol) antibody, the PEGylated insulin-like-growth-factor and the digoxygenylated insulin-like-growth-factor-binding-protein-4 is incubated with an anti-digoxygenin antibody, which is conjugated to horseradish peroxidase, and a third complex is formed. The third complex consists of the solid-phase conjugated anti-(polyethylene glycol) antibody, the thereto bound PEGylated insulin-like-growth-factor, the thereto bound digoxygenylated insulin-like-growth-factor-binding-protein-4, and the thereto bound anti-digoxygenin antibody conjugated to horseradish peroxidase. The third incubation step in one embodiment is of from 0.5 hours to 5 hours, e.g. about one hour. After the third incubation step the solid phase is optionally washed with a buffered solution.

In the fourth incubation step the complex consisting of the solid phase conjugated anti-(polyethylene glycol) antibody, the PEGylated insulin-like-growth-factor, the digoxygenylated insulin-like-growth-factor-binding-protein-4 and the anti-digoxygenin antibody conjugated to horseradish peroxidase is incubated with 2,2'-azino-bis-3-ethylbenzthiazoline-6-sulphonic acid (ABTS), a substrate for the enzyme horseradish peroxidase, which is converted by the enzyme to a colored product with an absorbance maximum at 405 nm. The concentration of the colored compound is proportional to the amount of the horseradish peroxidase and, thus, to the amount of the PEGylated insulin-like-growth-factor in the analyzed sample. A quantitative determination is therefore possible, if at least two reference samples with known PEGylated insulin-like-growth-factor concentration are analyzed, a smoothing function/calibration curve is determined and therewith the amount of PEGylated insulin-like-growth-factor is calculated.

The fourth incubation step is in one embodiment stopped, when the optical density (OD) of the solution at 405 nm reduced by the optical density of the solution at 490 nm (reference wavelength, blank) is of from 1.9 to 2.1. In another embodiment the fourth incubation step is of from 5 to 15 minutes, in a further embodiment of from 8 to 12 minutes.

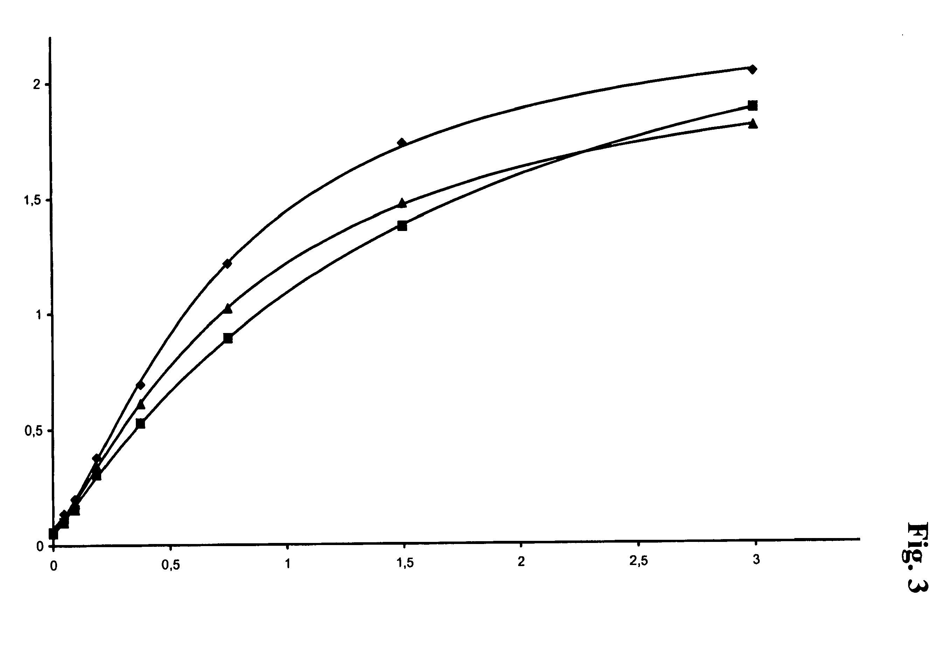

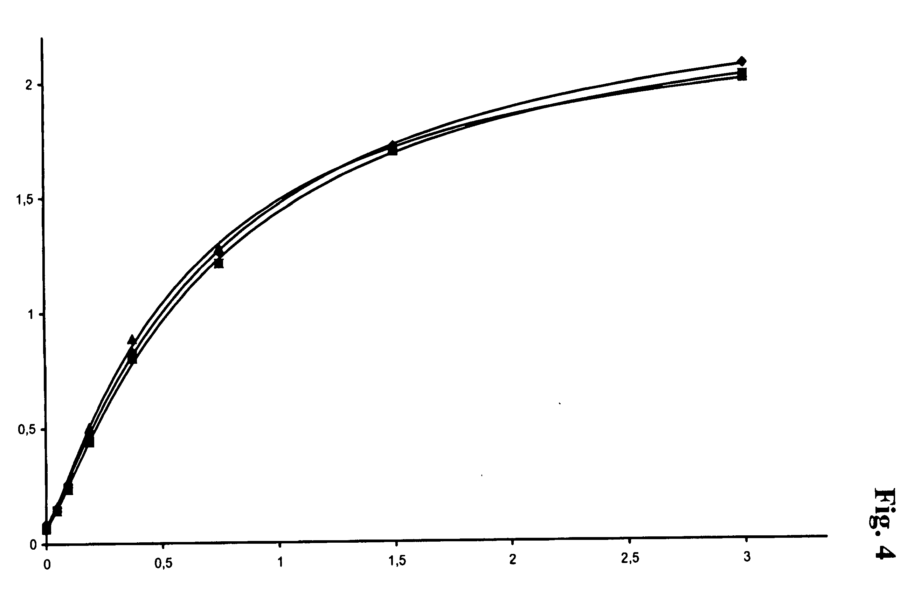

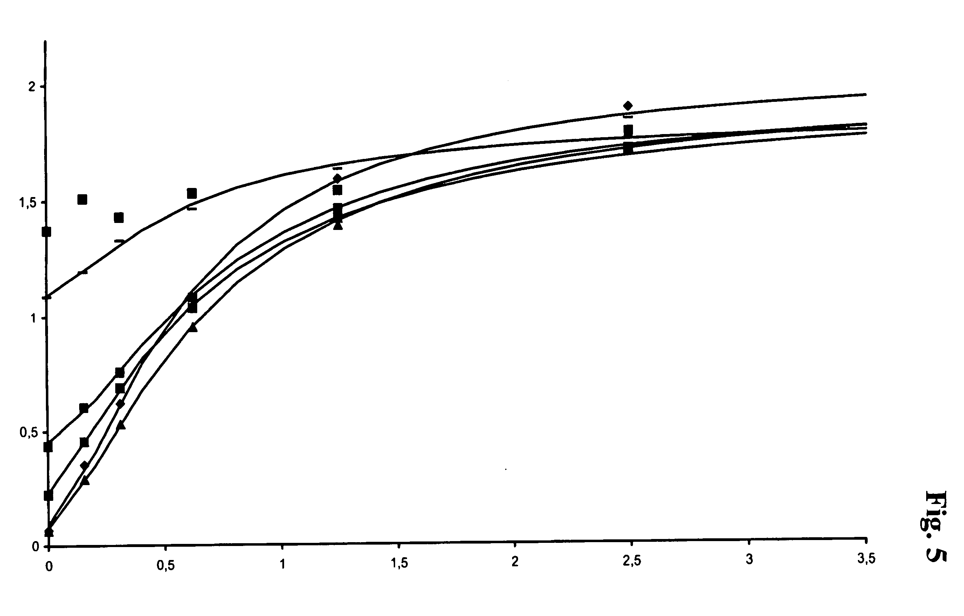

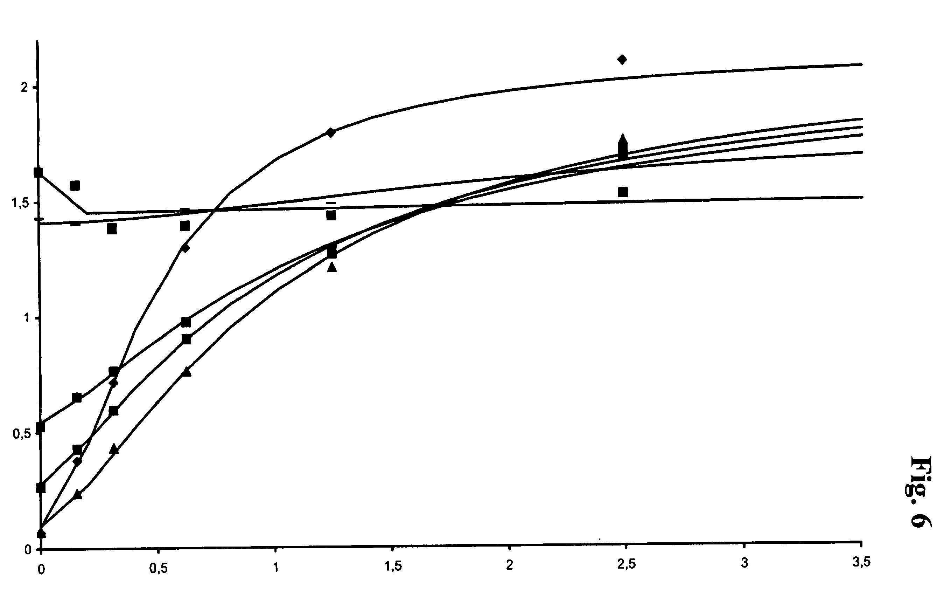

It has been found that some parameters in the immunoassay have to be carefully chosen. One of these parameters is the incubation time of the second incubation step of the complex consisting of the solid phase conjugated anti-(polyethylene glycol) antibody and the PEGylated insulin-like-growth-factor with the digoxygenylated insulin-like-growth-factor-binding-protein. It has been found that a longer incubation time results in an assay having a reduced susceptibility e.g. for other components of human serum or by competing insulin-like-growth-factor-binding-proteins (Figure 3 and 4). Therefore, in one embodiment is the incubation time in the second incubation step of the assay according to the invention 12 to 24 hours. A further parameter is the incubation temperature in the second incubation step. It has been found that an incubation temperature in the second incubation step of 20 to 25 °C, i.e. room temperature (Figure 5), is beneficial when compared to low temperature, e.g. 4 °C (Figure 6). A further parameter that has to be considered is the concentration of the employed digoxygenylated insulin-like-growth-factor-binding-protein. It has been found that said concentration has to be 5.0 µg/ml or lower. In one embodiment the concentration of the digoxygenylated insulin-like-growth-factor-binding-protein is from 0.1 µg/ml to 5.0 µg/ml, in a further embodiment from 0.1 µg/ml to 1.0 µg/ml.

It has to be pointed out that if the provided sample does not contain PEGylated insulin-like-growth-factor no complex is formed in the second incubation step and, thus, no colored product is formed in the fourth incubation step.

The third aspect of the current invention is a method for the quantitative determination of the amount of PEGylated insulin-like-growth-factor in a sample comprising the following steps:
a) providing a sample to be analyzed,
b) providing reference samples containing known amounts of PEGylated insulin-like-growth-factor,
c) incubating separately an anti-(polyethylene glycol) antibody conjugated to a solid phase with each of said sample and at least two reference samples containing different amounts of PEGylated insulin-like-growth-factor to form an anti-(polyethylene glycol) antibody/PEGylated insulin-like-growth-factor-complex,
d) incubating said complex formed in c) in each of the sample and reference samples with digoxygenylated insulin-like-growth-factor-binding-protein-4 to form a second complex comprising the complex formed in c), whereby the incubating with digoxygenylated insulin-like-growth-factor-binding-protein-4 is of from 12 to 20 hours,
e) incubating said complex formed in d) in each of the sample and reference samples with a horseradish peroxidase conjugated anti-digoxygenin antibody to form a third complex comprising the complex formed in d),
f) incubating the complex formed in e) in each of the sample and reference samples with ABTS for 5 to 15 minutes and determining the amount of the formed colored product,
g) quantitatively determining the amount of PEGylated insulin-like-growth-factor in said sample based on a calibration curve or smoothing function calculated based on the amount of the formed colored product in the reference samples.

The fourth aspect of the current invention is the use of the method according to the invention for the follow-up of a patient to whom PEGylated insulin-like-growth-factor has been administered.

The following examples, sequence listing and figures are provided to aid the understanding of the present invention, the true scope of which is set forth in the appended claims.

### Description of the Figures

- **Figure 1**: Scheme of the immunoassay according to the invention exemplified with insulin-like-growth-factor I and insulin-like growth-factor-binding-protein-4; 1: Streptavidin coated microtiter plate, 2: monoclonal biotinylated anti-(polyethylene glycol) antibody, 3: PEGylated insulin-like-growth-factor I, 4: digoxygenylated insulin-like-growth-factor-binding-protein-4, 5: monoclonal horseradish-peroxidase conjugated anti-digoxygenin antibody.

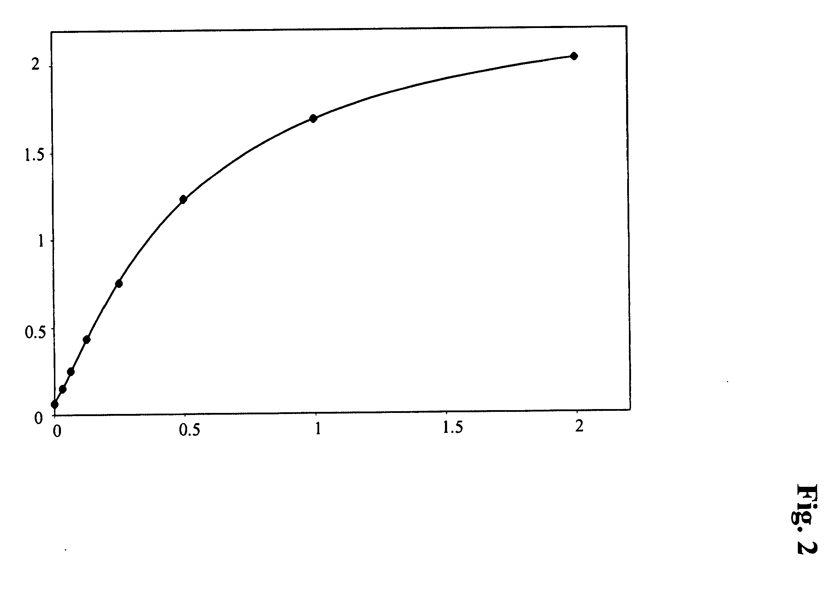

- **Figure 2**: Standard curve obtained with reference samples (Example 2); X-axis: concentration PEGylated insulin-like-growth-factor in ng/ml, Y-axis: mean absorption signal.
- **Figure 3**: Standard curves obtained with samples i) spiked with reference amounts of PEGylated insulin-like-growth-factor (diamond), ii) spiked with 5 % (v/v) human serum and reference amounts of PEGylated insulin-like-growth-factor (triangle), and iii) spiked with 10 ng/ml insulin-like-growth-factor-binding-protein-4, 5 % (v/v) human serum and reference amounts of PEGylated insulin-like-growth-factor (square); X-axis: concentration of PEGylated insulin-like-growth-factor in ng/ml, Y-axis: mean absorption signal; assay conditions: anti-(polyethylene glycol) antibody of IgM class, digoxygenylated insulin-like-growth-factor-binding-protein-4 at a concentration of 0.1 µg/ml, anti-digoxygenin antibody-horseradish peroxidase-conjugate at 50 mU/ml, all incubation times: 1 hour, room temperature, PEGylated insulin-like-growth-factor is a mixture of N-terminally PEGylated and at position 68 PEGylated protein.
- **Figure 4**: Standard curves obtained with samples i) spiked with reference amounts of PEGylated insulin-like-growth-factor (diamond), ii) spiked with 5 % (v/v) human serum and reference amounts of PEGylated insulin-like-growth-factor (triangle), and iii) spiked with 10 ng/ml insulin-like-growth-factor-binding-protein-4, 5 % (v/v) human serum and reference amounts of PEGylated insulin-like-growth-factor (square); X-axis: concentration of PEGylated insulin-like-growth-factor in ng/ml, Y-axis: mean absorption signal; assay conditions: anti-(polyethylene glycol) antibody of IgM class, digoxygenylated insulin-like-growth-factor-binding-protein-4 at a concentration of 0.1 µg/ml, anti-digoxygenin antibody-horseradish peroxidase-conjugate at 50 mU/ml, all incubation times: 1 hour except for the incubation with digoxygenylated insulin-like-growth-factor-binding-protein-4, which is 20 hours, room temperature, PEGylated insulin-like-growth-factor is a mixture of N-terminally PEGylated and at position 68 PEGylated protein.
- **Figure 5**: Standard curves obtained with samples i) spiked with reference amounts of PEGylated insulin-like-growth-factor and a concentration of digoxygenylated insulin-like-growth-factor-binding-protein-4 of 0.1 µg/ml (diamond), ii) spiked with reference amounts of PEGylated insulin-like-growth-factor, 20 ng/ml insulin-like-growth-factor-binding-protein-4 and a concentration of digoxygenylated insulin-like-growth-factor-binding-protein-4 of 0.1 µg/ml (triangle); iii) spiked with reference amounts of PEGylated insulin-like-growth-factor, 20 ng/ml insulin-like-growth-factor-binding-protein-4 and a concentration of digoxygenylated insulin-like-growth-factor-binding-protein-4 of 0.5 µg/ml (square); iv) spiked with reference amounts of PEGylated insulin-like-growth-factor, 20 ng/ml insulin-like-growth-factor-binding-protein-4 and a concentration of digoxygenylated insulin-like-growth-factor-binding-protein-4 of 1.0 µg/ml (small square); v) spiked with reference amounts of PEGylated insulin-like-growth-factor, 20 ng/ml insulin-like-growth-factor-binding-protein-4 and a concentration of digoxygenylated insulin-like-growth-factor-binding-protein-4 of 5.0 µg/ml (dashes); vi) spiked with reference amounts of PEGylated insulin-like-growth-factor, 20 ng/ml insulin-like- growth-factor-binding-protein-4 and a concentration of digoxygenylated insulin-like-growth-factor-binding-protein-4 of 10.0 µg/ml (small triangle); X-axis: concentration of PEGylated insulin-like-growth-factor in ng/ml, Y-axis: mean absorption signal; assay conditions: anti-(polyethylene glycol) antibody of IgM class, anti-digoxygenin antibody-horseradish peroxidase-conjugate at 50 mU/ml, all incubation times: 1 hour except for the incubation with digoxygenylated insulin-like-growth-factor-binding-protein-4, which is 20 hours, room temperature, PEGylated insulin-like-growth-factor is a mixture of N-terminally PEGylated and at position 68 PEGylated protein.
- **Figure 6**: Standard curves obtained with samples i) spiked with reference amounts of PEGylated insulin-like-growth-factor and a concentration of digoxygenylated insulin-like-growth-factor-binding-protein-4 of 0.1 µg/ml (diamond), ii) spiked with reference amounts of PEGylated insulin-like-growth-factor, 20 ng/ml insulin-like-growth-factor-binding-protein-4 and a concentration of digoxygenylated insulin-like-growth-factor-binding-protein-4 of 0.1 µg/ml (triangle); iii) spiked with reference amounts of PEGylated insulin-like-growth-factor, 20 ng/ml insulin-like-growth-factor-binding-protein-4 and a concentration of digoxygenylated insulin-like-growth-factor-binding-protein-4 of 0.5 µg/ml (square); iv) spiked with reference amounts of PEGylated insulin-like-growth-factor, 20 ng/ml insulin-like-growth-factor-binding-protein-4 and a concentration of digoxygenylated insulin-like-growth-factor-binding-protein-4 of 1.0 µg/ml (small square); v) spiked with reference amounts of PEGylated insulin-like-growth-factor, 20 ng/ml insulin-like-growth-factor-binding-protein-4 and a concentration of digoxygenylated insulin-like-growth-factor-binding-protein-4 of 5.0 µg/ml (dashes); vi) spiked with reference amounts of PEGylated insulin-like-growth-factor, 20 ng/ml insulin-like-growth-factor-binding-protein-4 and a concentration of digoxygenylated insulin-like-growth-factor-binding-protein-4 of 10.0 µg/ml (small triangle); X-axis: concentration of PEGylated insulin-like-growth-factor in ng/ml, Y-axis: mean absorption signal; assay conditions: anti-(polyethylene glycol) antibody of IgM class, anti-digoxygenin antibody-horseradish peroxidase-conjugate at 50 mU/ml, all incubation times: 1 hour except for the incubation with digoxygenylated insulin-like-growth-factor-binding-protein-4, which is 20 hours, 4 °C, PEGylated insulin-like-growth-factor is a mixture of N-terminally PEGylated and at position 68 PEGylated protein.

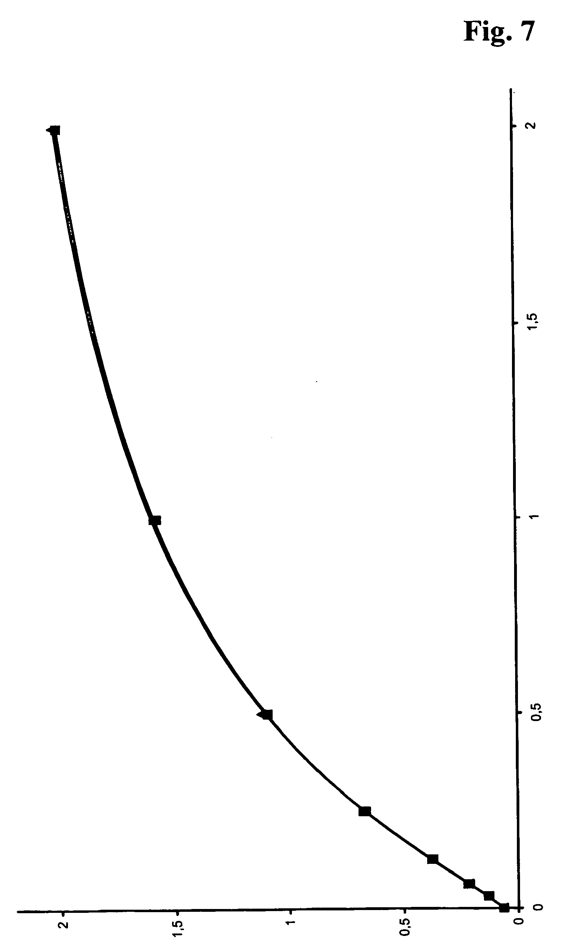

- **Figure 7**: Comparison of standard curves obtained with samples i) spiked with reference amounts of PEGylated insulin-like-growth-factor and 5 % (v/v) human serum (triangle), ii) spiked with reference amounts of PEGylated insulin-like-growth-factor and 5 % (v/v) mouse serum (triangle); X-axis: concentration of PEGylated insulin-like-growth-factor in ng/ml, Y-axis: mean absorption signal; assay conditions: anti-(polyethylene glycol) antibody of IgM class, anti-digoxygenin antibody-horseradish peroxidase-conjugate at 25 mU/ml, all incubation times: 1 hour except for the incubation with digoxygenylated insulin-like-growth-factor-binding-protein-4, which is 20 hours, room temperature, PEGylated insulin-like-growth-factor is a mixture of N-terminally PEGylated and at position 68 PEGylated protein.

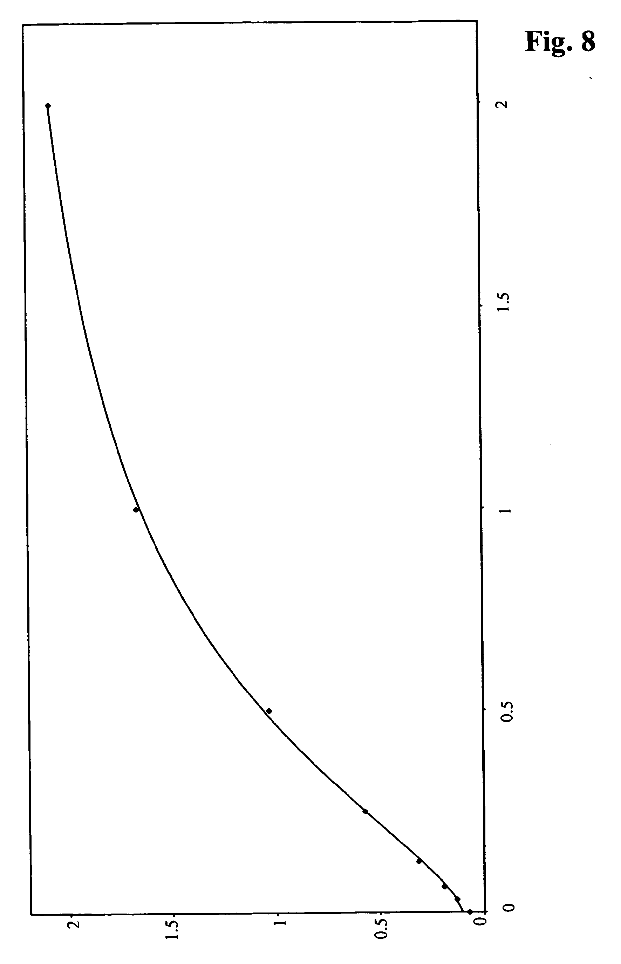

- **Figure 8**: Standard curve obtained with reference samples spiked with reference amounts of PEGylated insulin-like-growth-factor and 5 % (v/v) human plasma; X-axis: concentration PEGylated insulin-like growth-factor in ng/ml, Y-axis: mean absorption signal.

### Description of the Sequences

- **SEQ ID NO: 1**: Amino acid sequence of human insulin-like-growth-factor I (amino acids 49 to 118 of Swiss-Prot ID P01343).
- **SEQ ID NO: 2**: Amino acid sequence of human insulin-like-growth-factor II (amino acids 25 to 91 of Swiss-Prot ID P01344).
- **SEQ ID NO: 3**: Amino acid sequence of human insulin-like-growth-factor-binding-protein-4 (amino acids 22 to 258 of Swiss-Prot ID P22692).

### Example 1:

### Preparation of anti-(polyethylene glycol) antibody conjugated to a microtiter plate

A solution of a biotinylated anti-(polyethylene glycol) antibody with a final antibody concentration of 2 µg/ml was added to the wells of a 96-well Streptavidin-coated microtiter plate (MicroCoat) with 100 µl to each well. Afterwards the solution is incubated at room temperature at 500 rpm for one hour. Thereafter the solution is discarded and the wells are washed three times each with 300 µl washing buffer (1x PBS (phosphate buffered saline) supplemented with 0.05 % (w/v) n-octylglycosid).

### Example 2:

### Preparation of samples

### a) Standard sample

A stock solution of PEGylated insulin-like-growth-factor I (for preparation of PEGylated insulin-like-growth-factor I see e.g. WO 2006/066891) with a concentration of 2 ng/ml in PBS buffer (phosphate buffered saline) supplement with 0.5 % (w/v) bovine plasma albumin 1 was prepared. The stock solution was diluted to the following concentration:

| |
|---|
| 2.00 ng/ml |
| 1.00 ng/ml |
| 0.50 ng/ml |
| 0.25 ng/ml |
| 0.13 ng/ml |
| 0.06 ng/ml |
| 0.03 ng/ml |
| 0.00 ng/ml |

### b) Reference sample with serum or plasma

A stock solution of PEGylated insulin-like-growth-factor I (for preparation of PEGylated insulin-like-growth-factor I see e.g. WO 2006/066891) with a concentration of 2 ng/ml in 5 % pooled blank mouse serum or 5 % pooled blank human serum or 5 % pooled blank human plasma in PBS buffer (phosphate buffered saline) supplement with 0.5 % (w/v) bovine plasma albumin 1 was prepared. The stock solution was diluted to the following concentration:

| |
|---|
| 2.00 ng/ml |
| 1.00 ng/ml |
| 0.50 ng/ml |
| 0.25 ng/ml |
| 0.13 ng/ml |
| 0.06 ng/ml |
| 0.03 ng/ml |
| 0.00 ng/ml |

### c) Test sample

The unknown test serum sample is diluted 1:20 with 5 % pooled blank mouse serum in phosphate buffered saline supplemented with 0.5 % (w/v) bovine plasma albumin 1.

### Example 3:

### Immunoassay

To the wells of a microtiter plate obtained according to Example 1 were added 100 µl of each reference and test sample in duplicate. The wells were incubated for one hour with shaking at 500 rpm. Afterwards the solution is discarded and each well is washed three times each with 300 µl phosphate buffered saline supplemented with 0.05 % (w/v) n-octylglycosid. Thereafter 100 µl of a solution of digoxygenylated insulin-like-growth-factor-binding-protein-4 at 100 ng/ml was added to each well and incubated for 12-24 hours, preferably 20 hours, with shaking at 500 rpm. Afterwards the solution was discarded and each well was washed three times each with 300 µl phosphate buffered saline supplemented with 0.05 % (w/v) n-octylglycosid. Thereafter 100 µl of a solution of an anti-digoxygenin antibody conjugated to horseradish peroxidase with a final concentration of 50 mU/ml was added to each well and incubated for one hour with shaking at 500 rpm. Afterwards the solution in the wells was discarded and each well was washed three times each with 300 µl phosphate buffered saline supplemented with 0.05 % (w/v) n-octylglycosid. Thereafter 100 µl of an ABTS solution was added to each well. The reaction was stopped when the highest standard solution of 2 ng/ml has reached an OD value of 1.9 - 2.0. This normally requires between 5 to 15 minutes. The OD of the standard and test samples was measured at 405 nm and 490 nm. A standard curve of the reference standards was obtained using a 4-Parameter fit program. With the standard curve the amount of PEGylated insulin-like-growth-factor I in the test samples was calculated. The lower limit of detection and lower limit of quantification have been calculated to be at 20 pg/ml and 31 pg/ml, respectively. All steps were carried out at room temperature.

**Table 1: Typical results of the positive control.**

| **Sample No. 1** | **Sample No. 2** | **Sample No. 3** | **Sample concentration** | **mean** | **STDEV** | **CV [%]** |
|---|---|---|---|---|---|---|
| 2.0350 | 2.0550 | 2.0020 | 2.00 ng/ml | 2.0307 | 0.0268 | 1.3% |
| 1.6600 | 1.6640 | 1.7330 | 1.00 ng/ml | 1.6857 | 0.0410 | 2.4% |
| 1.2090 | 1.2520 | 1.2240 | 0.50 ng/ml | 1.2283 | 0.0218 | 1.8% |
| 0.7570 | 0.7340 | 0.7660 | 0.25 ng/mt | 0.7523 | 0.0165 | 2.2% |
| 0.4390 | 0.4280 | 0.4360 | 0.13 ng/ml | 0.4343 | 0.0057 | 1.3% |
| 0.2500 | 0.2520 | 0.2430 | 0.06 ng/ml | 0.2483 | 0.0047 | 1.9% |
| 0.1410 | 0.1590 | 0.1490 | 0.03 ng/ml | 0.1497 | 0.0090 | 6.0% |
| 0.0590 | 0.0620 | 0.0660 | 0.00 ng/ml | 0.0623 | 0.0035 | 5.6% |

### Example 4

### Biotinylation of anti-(polyethylene glycol) antibody

An antibody against polyethylene glycol was dialyzed against buffer (100 mM potassium phosphate buffer, pH 8.5). Afterwards the solution was adjusted to a protein concentration of 10 mg/ml. D-biotinoyl-aminocaproic acid-N-hydroxysuccinimide ester was dissolved in DMSO and added to the antibody solution in a molar ratio of 1:5. After 60 minutes the reaction was stopped by adding L-lysine. The surplus of the labeling reagent was removed by dialysis against 25 mM potassium phosphate buffer supplemented with 150 mM NaCl, pH 7.5.

### Example 5

### Digoxigenylation of insulin-like-growth-factor-binding-protein

Insulin-like-growth-factor-binding-protein was dialyzed against digoxygenylation buffer (100 mM potassium phosphate buffer, pH 8.5). Afterwards the solution was adjusted to a protein concentration of 10 mg/ml. Digoxigenin 3-O-methylcarbonyl-ε-aminocaproic acid-N-hydroxysuccinimide ester was dissolved in DMSO and added to the antibody solution in a molar ratio of 1:5. After 60 minutes the reaction was stopped by adding L-lysine. The surplus of labeling reagent was removed by dialysis against 25 mM potassium phosphate buffer supplemented with 150 mM NaCl, pH 7.5.

## Claims

1. Immunoassay for the detection of PEGylated insulin-like-growth-factor comprising a capture antibody and a tracer antibody, **characterized in that**
a) said capture antibody is a monoclonal anti-(polyethylene glycol) antibody,
b) said PEGylated insulin-like-growth-factor is detected as a complex with a digoxygenylated insulin-like-growth-factor-binding-protein,
c) said tracer antibody is a monoclonal anti-digoxygenin antibody,
whereby the incubation of said PEGylated insulin-like-growth-factor and said digoxygenylated insulin-like-growth-factor-binding-protein is for 12 to 24 hours at room temperature with a concentration of said digoxygenylated insulin-like-growth-factor-binding-protein of 5.0 µg/ml or less.

2. Immunoassay according to claim 1, **characterized in that**
a) said anti-(polyethylene glycol) antibody is conjugated to a solid phase, and
b) said anti-digoxygenin antibody is conjugated to a detectable label.

3. Immunoassay according to claim 2, **characterized in that** said detectable label is selected from enzymes, antigens, fluorescent groups, chemoluminescent groups, electrochemiluminescent groups, and metal-chelate complexes.

4. Immunoassay according to any one of the preceding claims, **characterized in that** said PEGylated insulin-like-growth-factor is a PEGylated insulin-like-growth-factor I or a PEGylated variant thereof.

5. Immunoassay according to any one of the preceding claims, **characterized in that** said insulin-like-growth-factor-binding-protein is insulin-like-growth-factor-binding-protein-4.

6. Immunoassay according to any one of the preceding claims, **characterized in that** the incubation of said PEGylated insulin-like-growth-factor and said digoxygenylated insulin-like-growth-factor-binding-protein is for 18 to 22 hours.

7. Immunoassay according to any one of the preceding claims, **characterized in that** the incubation step of said PEGylated insulin-like-growth-factor and said digoxygenylated insulin-like-growth-factor-binding-protein is with a concentration of said digoxygenylated insulin-like-growth-factor-binding-protein of 0.1 µg/ml to 5.0 µg/ml.

8. Method for the determination of PEGylated insulin-like-growth-factor in a sample comprising the following steps:
a) providing a sample to be analyzed,
b) incubating an anti-(polyethylene glycol) antibody conjugated to a solid phase with said sample to form an anti-(polyethylene glycol) antibody/PEGylated insulin-like-growth-factor-complex,
c) incubating said complex formed in b) with digoxygenylated insulin-like-growth-factor-binding-protein-4 to form a complex comprising the complex formed in b) at room temperature for 12 to 24 hours with a concentration of said digoxygenylated insulin-like-growth-factor-binding-protein-4 of 5.0 µg/ml or less,
d) incubating said complex formed in c) with a horseradish peroxidase conjugated anti-digoxygenin antibody to form a complex comprising the complex formed in c),
e) determining PEGylated insulin-like-growth-factor by incubating the complex formed in d) with ABTS and by the formation of a colored product.

9. Method according to claim 8, **characterized in that** after steps b), c), and/or d) a washing step is carried out.

10. Method according to claim 8 or 9, **characterized in that** said PEGylated insulin-like-growth-factor is PEGylated insulin-like-growth-factor-I or a PEGylated variant thereof.

11. Method according to any one of claims 8 to 10, **characterized in that** the incubating of said PEGylated insulin-like-growth-factor and said digoxygenylated insulin-like-growth-factor-binding-protein-4 is for 18 to 22 hours.

12. Method according to any one of the claims 8 to 11, **characterized in that** the incubating of said PEGylated insulin-like-growth-factor and said digoxygenylated insulin-like-growth-factor-binding-protein-4 is with a concentration of said digoxygenylated insulin-like-growth-factor-binding-protein-4 of 0.1 µg/ml to 5.0 µg/ml.

13. Use of a method according to claim 8 for the follow up of a patient that is administered PEGylated insulin-like growth-factor I or a PEGylated variant thereof.

14. Method for the quantitative determination of the amount of PEGylated insulin-like-growth-factor in a sample comprising the following steps:
a) providing a sample to be analyzed,
b) providing a reference sample containing a defined amount of PEGylated insulin-like-growth-factor,
c) incubating an anti-(polyethylene glycol) antibody conjugated to a solid phase each with said sample and at least two reference samples containing different amounts of PEGylated insulin-like-growth-factor to form an anti-(polyethylene glycol) antibody/PEGylated insulin-like-growth-factor-complex,
d) incubating said complex formed in c) in each of the sample and reference samples with digoxygenylated insulin-like-growth-factor-binding-protein-4 to form a second complex comprising the complex formed in c), whereby the incubating with digoxygenylated insulin-like-growth-factor-binding-protein-4 is for 12 to 24 hours at room temperature with a concentration of said digoxygenylated insulin-like-growth-factor-binding-protein-4 of 5.0 µg/ml or less,
e) incubating said complex formed in d) in each of the sample and reference samples with a horseradish peroxidase conjugated anti-digoxygenin antibody to form a third complex comprising the complex formed in d),
f) incubating the complex formed in e) in each of the sample and reference samples with ABTS for 5 to 15 minutes and determining the amount of the formed colored product,
g) quantitatively determining the amount of PEGylated insulin-like growth-factor in said sample based on a calibration curve calculated based on the amount of the formed colored product in the reference samples.

15. Method according to claim 14, **characterized in that** in step d) said incubating is for 18 to 22 hours.

## Patentansprüche

1. Immunassay zur Detektion von PEGyliertern insulinähnlichem Wachstumsfaktor, umfassend einen Fang-Antikörper und einen Tracer-Antikörper, **dadurch gekennzeichnet, dass**
a) der Fang-Antikörper ein monoklonaler anti-(Polyethylenglycol)-Antikörper ist,
b) der PEGylierte insulinähnliche Wachstumsfaktor als ein Komplex mit einem digoxigenyliertem insulinähnlichem Wachstumsfaktor-Bindeprotein detektiert wird,
c) der Tracer-Antikörper ein monoklonaler anti-Digoxigenin-Antikörper ist,
wobei die Inkubation des PEGylierten insulinähnlichen Wachstumsfaktors und des digoxigenylierten insulinähnlichen Wachstumsfaktor-Bindeproteins für 12 bis 24 Stunden bei Raumtemperatur mit einer Konzentration des digoxigenylierten insulinähnlichen Wachstumsfaktor-Bindeproteins von 5,0 µg/ml oder weniger erfolgt.

2. Immunassay nach Anspruch 1, **dadurch gekennzeichnet, dass**
a) der anti-(Polyethylenglycol)-Antikörper an eine Festphase konjugiert ist und
b) der anti-Digoxigenin-Antikörper an eine detektierbare Markierung konjugiert ist.

3. Immunassay nach Anspruch 2, **dadurch gekennzeichnet, dass** die detektierbare Markierung ausgewählt ist aus Enzymen, Antigenen, Fluorenszenzgruppen, Chemilumineszenzgruppen, Elektrochemilumineszenzgruppen und Metall-Chelat-Komplexen.

4. Immunassay nach einem der vorherigen Ansprüche, **dadurch gekennzeichnet, dass** der PEGylierte insulinähnliche Wachstumsfaktor ein PEGylierter insulinähnlicher Wachstumsfaktor I oder eine PEGylierte Variante davon ist.

5. Immunassay nach einem der vorherigen Ansprüche, **dadurch gekennzeichnet, dass** das insulinähnlicher Wachstumsfaktor-Bindeprotein insulinähnlicher Wachstumsfaktor-Bindeprotein-4 ist.

6. Immunassay nach einem der vorherigen Ansprüche, **dadurch gekennzeichnet, dass** die Inkubation des PEGylierten insulinähnlichen Wachstumsfaktors und des digoxigenylierten insulinähnlichen Wachstumsfaktor-Bindeproteins für 18 bis 22 Stunden erfolgt.

7. Immunassay nach einem der vorherigen Ansprüche, **dadurch gekennzeichnet, dass** der Inkubationsschritt des PEGylierten insulinähnlichen Wachstumsfaktors und des digoxigenylierten insulinähnlichen Wachstumsfaktor-Bindeproteins mit einer Konzentration des digoxigenylierten insulinähnlichen Wachstumsfaktor-Bindeproteins von 0,1 µg/ml bis 5,0 µg/ml erfolgt.

8. Verfahren zur Bestimmung von PEGyliertem insulinähnlichem Wachstumsfaktor in einer Probe, umfassend die folgenden Schritte:
a) Bereitstellen einer zu analysierenden Probe,
b) Inkubieren eines anti-(Polyethylenglycol)-Antikörpers, der an eine Festphase konjugiert ist, mit der Probe, um einen anti-(Polyethylenglycol)-Antikörper/PEGylierter insulinähnlicher Wachstumsfaktor-Komplex zu bilden.
c) Inkubieren des in b) gebildeten Komplexes mit digoxigenyliertem insulinähnlichem Wachstumsfaktor-Bindeprotein-4, um einen Komplex zu bilden, umfassend den in b) gebildeten Komplex, bei Raumtemperatur für 12 bis 24 Stunden mit einer Konzentration des digoxigenylierten insulinähnlichen Wachstumsfaktor-Bindeproteins-4 von 5,0 µg/ml oder weniger,
d) Inkubieren des in c) gebildeten Komplexes mit einem Meerettich-Peroxidase konjugiertem anti-Digoxigenin-Antikörper, um einen Komplex zu bilden, umfassend den in c) gebildeten Komplex,
e) Bestimmen von PEGyliertem insulinähnlichem Wachstumsfaktor durch Inkubation des in d) gebildeten Komplexes mit ABTS und durch die Bildung eines gefärbten Produkts.

9. Verfahren nach Anspruch 8, **dadurch gekennzeichnet, dass** nach den Schritten b), c) und/oder d) ein Waschschritt durchgeführt wird.

10. Verfahren nach Anspruch 8 oder 9, **dadurch gekennzeichnet, dass** der PEGylierte insulinähnliche Wachstumsfaktor PEGylierter insulinähnlicher Wachstumsfaktor-I oder eine PEGylierte Variante davon ist.

11. Verfahren nach einem der Ansprüche 8 bis 10, **dadurch gekennzeichnet, dass** die Inkubation des PEGylierten insulinähnlichen Wachstumsfaktors und des digoxigenylierten insulinähnlichen Wachstumsfaktor-Bindeproteins-4 für 18 bis 22 Stunden erfolgt.

12. Verfahren nach einem der Ansprüche 8 bis 11, **dadurch gekennzeichnet, dass** die Inkubation des PEGylierten insulinähnlichen Wachstumsfaktors und des digoxigenylierten insulinähnlichen Wachstumsfaktor-Bindeproteins-4 mit einer Konzentration des digoxigenylierten insulinähnlichen Wachstumsfaktor-Bindeproteins-4 von 0,1 µg/ml bis 5,0 µg/ml erfolgt.

13. Verwendung eines Verfahrens nach Anspruch 8 für die Nachsorge eines Patienten, dem PEGylierter insulinähnlicher Wachstumsfaktor-1 oder eine PEGylierte Variante davon verabreicht wurde.

14. Verfahren zur quantitativen Bestimmung der Menge an PEGyliertem insulinähnlichem Wachstumsfaktor in einer Probe, umfassend die folgenden Schritte:
a) Bereitstellen einer zu analysierenden Probe,
b) Bereitstellen einer Referenzprobe, die eine bestimmte Menge an PEGyliertem insulinähnlichem Wachstumsfaktor enthält,
c) Inkubieren eines anti-(Polyethylenglycol)-Antikörpers, der an eine Festphase konjugiert ist, jeweils mit der Probe und mindestens zwei Referenzproben, die unterschiedliche Mengen an PEGyliertem insulinähnlichem Wachstumsfaktor enthalten, um einen anti-(Polyethylenglycol)-Antikörper/PEGylierter insulinähnlicher Wachstumsfaktor-Komplex zu bilden,
d) Inkubieren des in c) gebildeten Komplexes jeweils in der Probe und den Referenzproben mit digoxigenyliertem insulinähnlichem Wachstumsfaktor-Bindeprotein-4, um einen zweiten Komplex zu bilden, umfassend den in c) gebildeten Komplex, wobei die Inkubation mit digoxigenyliertem insulinähnlichem Wachstumsfaktor-Bindeprotein-4 bei Raumtemperatur für 12 bis 24 Stunden mit einer Konzentration des digoxigenylierten insulinähnlichen Wachstumsfaktor-Bindeproteins-4 von 5,0 µg/ml oder weniger erfolgt,
e) Inkubieren des in d) gebildeten Komplexes jeweils in der Probe und den Referenzproben mit einem Meerettich-Peroxidase konjugiertem anti-Digoxigenin-Antikörper, um einen dritten Komplex zu bilden, umfassend den in d) gebildeten Komplex,
f) Inkubieren des in e) gebildeten Komplexes jeweils in der Probe und den Referenzproben mit ABTS für 5 bis 15 Minuten und Bestimmen der Menge des gebildeten gefärbten Produkts,
g) quantitativ Bestimmen der Menge an PEGyliertem insulinähnlichem Wachstumsfaktor in der Probe, basierend auf einer Eichkurve, die basierend auf der Menge des gebildeten gefärbten Produkts in den Referenzproben berechnet wurde.

15. Verfahren nach Anspruch 14, **dadurch gekennzeichnet, dass** in Schritt d) die Inkubation for 18 bis 22 Stunden erfolgt.

## Revendications

1. Analyse immunologique pour la détection du facteur de croissance analogue à l'insuline pégylé, comprenant un anticorps de capture et un anticorps traceur, **caractérisée en ce que**
a) ledit anticorps de capture est un anticorps monoclonal anti-(polyéthylène-glycol),
b) ledit facteur de croissance analogue à l'insuline pégylé est détecté sous forme d'un complexe avec une protéine de liaison au facteur de croissance analogue à l'insuline
c) ledit anticorps traceur est un anticorps monoclonal anti-digoxygénine, l'incubation dudit facteur de croissance analogue à l'insuline pégylé et de ladite protéine de liaison au facteur de croissance analogue à l'insuline digoxygénylée s'effectuant pendant 12 à 24 heures à la température ambiante avec une concentration de ladite protéine de liaison au facteur de croissance analogue à l'insuline digoxygénylée d'au plus 5,0 µg/ml.

2. Analyse immunologique selon la revendication 1, **caractérisée en ce que**
a) ledit anticorps anti-(polyéthylèneglycol) est conjugué à une phase solide, et
b) ledit anticorps anti-digoxygénine est conjugué à un marqueur détectable.

3. Analyse immunologique selon la revendication 2, **caractérisée en ce que** ledit marqueur détectable est choisi parmi des enzymes, des antigène, des groupes fluorescents, des groupes chimilummescents, des groupes électrochimiluminescents et des complexes de chélates métalliques.

4. Analyse immunologique selon l'une quelconque des revendications précédente, **caractérisée en ce que** ledit facteur de croissance analogue à l'insuline pégylé est un facteur de croissance analogue à l'insuline I pégylé ou une de ses variantes pégylées.

5. Analyse immunologique selon l'une quelconque des revendications précédentes, **caractérisée en ce que** ladite protéine de liaison au facteur de croissance analogue à l'insuline est la protéine de liaison au facteur de croissance analogue à l'insuline 4.

6. Analyse immunologique selon l'une quelconque des revendications précédentes, **caractérisée en ce que** l'incubation dudit facteur de croissance analogue à l'insuline pégylé et de ladite protéine de liaison au facteur de croissance analogue à l'insuline digoxygénylée est effectuée pendant 18 à 22 heures.

7. Analyse immunologique selon l'une quelconque des revendications précédentes, **caractérisée en ce que** l'incubation dudit facteur de croissance analogues à l'insuline pégylé et de ladite protéine de liaison au facteur de croissance analogue à l'insuline digoxygénylée est effectuée avec une concentration de ladite protéine de liaison au facteur de croissance analogue à l'insuline digoxygénylée de 0.1 µg/ml à 5,0 µg/ml.

8. Procédé de détermination du facteur de croissance analogue à l'insuline pégylé dans un échantillon, comprenant les étapes suivantes;
a) fourniture d'un échantillon à analyser,
b) incubation d'un anticorps anti-(polyéthylèneglycol) conjugué à une phase solide avec ledit échantillon pour former un complexe anticorps anti-(polyéthylène-glycol)/facteur de croissance analogue à l'insuline pégylé,
c) incubation dudit complexe formé dans b) avec une protéine de liaison au facteur de croissance analogue à l'insuline 4 digoxygénylée pour former un complexe comprenant le complexe formé dans b) à la température ambiante pendant 12 à 24 heures avec une concentration de ladite protéine de liaison au facteur de croissance analogue à l'insuline 4 digoxygénylée d'au plus 5,0 µg/ml,
d) incubation dudit complexe formé dans c) avec un anticorps anti-digoxygénine conjugué à de la peroxydase de raifort pour former un complexe comprenant le complexe formé dans c),
e) détermination du facteur de croissance analogue à l'insuline pégylé par incubation du complexe formé dans d) avec de l'ABTS et formation d'un produit coloré.

9. Procédé selon la revendication 8, **caractérisé en ce que**, après les étapes b), c) et/ou d), on effectue une étape de lavage.

10. Procédé selon la revendication 8 ou la revendication 9, **caractérisé en ce que** ledit facteur de croissance analogue à l'insuline pégylé est le facteur de croissance analogue à l'insuline I pégylé ou une de ses variantes pégylées.

11. Procédé selon l'une quelconque des revendications 8 à 10, **caractérisé en ce que** l'incubation dudit facteur de croissance analogue à l'insuline pégylé et de ladite protéine de liaison au facteur de croissance analogue à l'insuline 4 digoxygénylée est effectuée pendant 18 à 22 heures.

12. Procédé selon l'une quelconque des revendications 8 à 11, **caractérisé en ce que** l'incubation dudit facteur de croissance analogue à l'insuline pégylé et de ladite protéine de liaison au facteur de croissance analogue à l'insuline 4 digoxygénylée est effectuée avec une concentration de ladite protéine de liaison au facteur de croissance analogue à l'insuline 4 digoxygénylée de 0,1 µg/ml à 5,0 µg/ml.

13. Utilisation d'un procédé selon la revendication 8 pour le suivi d'un patient auquel est administré du facteur de croissance analogue à l'insuline 1 pégylé ou une de ses variantes pégylées.

14. Procédé de détermination quantitative de la quantité de facteur de croissance analogue à l'insuline pégylé dans un échantillon, comprenant les étapes suivantes:
a) fourniture d'un échantillon à analyser,
b) fourniture d'un échantillon de référence contenant une quantité définie de facteur de croissance analogue à l'insuline pégylé,
c) incubation d'un anticorps anti-(polyéthylèneglycol) conjugué à une phase solide à chaque fois avec ledit échantillon et au moins deux échantillons de référence contenant différentes quantités de facteur de croissance analogue à l'insuline pégylé pour former un complexe anticorps anti-(polyéthylèneglycol)/facteur de croissance analogue à l'insuline pégylé,
d) incubation dudit complexe formé dans c) dans l'échantillon et chacun des échantillons de référence avec une protéine de liaison au facteur de croissance analogue à l'insuline 4 digoxygénylée pour former un deuxième complexe comprenant le complexe formé dans c), l'incubation avec la protéine de liaison au facteur de croissance analogue à l'insuline 4 digoxygénylée étant effectuée pendant 12 à 24 heures à la température ambiante avec une concentration de ladite protéine de liaison au facteur de croissance analogue à l'insuline 4 digoxygénylée d'au plus 5,0 µg/ml.
e) incubation dudit complexe formé dans d) dans l'échantillon et chacun des échantillons de référence avec un anticorps anti-digoxygénine conjugué à de la peroxydase de raifort pour former un troisième complexe comprenant le complexe formé dans d),
f) incubation du complexe formé dans e) dans l'échantillon et chacun des échantillons de référence avec de l'ABTS pendant 5 à 15 minutes et détermination de la quantité de produit coloré formé,
g) détermination quantitative de la quantité de facteur de croissance analogue à l'insuline pégylé dans ledit échantillon sur la base d'une courbe d'étalonnage calculée sur la base de la quantité de produit coloré formé dans les échantillons de référence.

15. Procédé selon la revendication 14, **caractérisé en ce que**, dans l'étape d), ladite incubation est effectuée pendant 18 à 22 heures.
